(19) Europäisches Patentamt
European Patent Office
Office européen des brevets

(11) **EP 3 110 330 B1**

(12) **EUROPÄISCHE PATENTSCHRIFT**

(45) Veröffentlichungstag und Bekanntmachung des Hinweises auf die Patenterteilung:
**31.03.2021 Patentblatt 2021/13**

(21) Anmeldenummer: **15707043.4**

(22) Anmeldetag: **26.02.2015**

(51) Int Cl.:
**A61B 5/11** *(2006.01)*     **F16F 15/22** *(2006.01)*

(86) Internationale Anmeldenummer:
**PCT/EP2015/000452**

(87) Internationale Veröffentlichungsnummer:
**WO 2015/128090 (03.09.2015 Gazette 2015/35)**

(54) **VORRICHTUNG ZUM REDUZIEREN VON TREMOR**

DEVICE FOR REDUCING TREMOR

DISPOSITIF DE RÉDUCTION DU TREMBLEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.02.2014 DE 102014002910**

(43) Veröffentlichungstag der Anmeldung:
**04.01.2017 Patentblatt 2017/01**

(73) Patentinhaber: **Fraunhofer-Gesellschaft zur Förderung der angewandten Forschung e.V.
80686 München (DE)**

(72) Erfinder:
• **VELTEN, Thomas
66507 Reifenberg (DE)**
• **KURZ, Christian
66292 Riegelsberg (DE)**

(74) Vertreter: **v. Bezold & Partner Patentanwälte - PartG mbB
Akademiestraße 7
80799 München (DE)**

(56) Entgegenhaltungen:
**WO-A2-01/80710     US-A1- 2003 006 357
US-B1- 6 234 045**

• **KALYANA C. VELUVOLU ET AL: "Estimation of Physiological Tremor from Accelerometers for Real-Time Applications", SENSORS, Bd. 11, Nr. 12, 7. März 2011 (2011-03-07), Seiten 3020-3036, XP55186189, ISSN: 1424-8220, DOI: 10.3390/s110303020 in der Anmeldung erwähnt**

**Beschreibung**

Die Erfindung betrifft eine am Körper tragbare Vorrichtung zum Reduzieren von Tremor eines Körperteils.

Dem Stand der Technik entsprechende Vorrichtungen werden zum Beispiel in US2003/006357A1, WO01/80710A2 und US6234045B1 beschrieben.

Als Tremor wird das unwillkürliche, sich rhythmisch wiederholende Zusammenziehen einander entgegenwirkender Muskelgruppen bezeichnet. Deutlich sichtbarer Tremor kann als ein Symptom verschiedener Erkrankungen auftreten. Der Tremor an sich kann das Leben des Patienten mehr oder minder stark beeinträchtigen, abhängig von Stärke, Erscheinungsform und betroffenen Körperregionen. Insbesondere ein die Hand betreffender ausgeprägter sogenannter Intensionstremor kann sehr behindernd sein, da viele Bewegungen des alltäglichen Lebens zielgerichtet sind und der Intensionstremor demzufolge alltägliche Vorgänge, welche eine ruhige Hand erfordern, wie Essen, Trinken oder Schreiben erschwert bis unmöglich macht.

Die Tremorfrequenz hängt von den unterschiedlichen Muskelsystemen und ggf. von den Krankheitsbildern ab. Es wird zwischen niederfrequentem, grobschlägigem Tremor, der seltener aufritt, dafür ausfahrender ist, mittelfrequentem Tremor und hochfrequenten, oft feinschlägigem Tremor, einer Art feinen Zitterns, unterschieden. Die Frequenzen liegen im ersten Fall unter 4 Hz, beim mittelfrequenten Tremor bei 4 bis 7 Hz und beim feinschlägigen Tremor bei bis zu 15 Hz. Bei der Parkinson-Krankheit liegt der Tremor in Ruhe vor und betrifft am häufigsten die Arme. Bei Bewegung der betroffenen Extremität verschwindet oder bessert sich der Tremor und erscheint nach kurzer Zeit wieder, wenn eine neue Ruheposition eingenommen wird. Er wird intensiviert durch geistigen Stress und verschwindet im Schlaf. Er ist von relativ langsamer Frequenz bei einer sehr genau definierten Frequenz mit einer Frequenz von vier bis sechs Hz, vgl. RP Meshack and KE Norman, A randomized controlled trial of the effects of weights on amplitude and frequency of postural hand tremor in people with Parkinson's disease, Clinical Rehabilitation 2002; 16: 481-492. Hochfrequente Zitterbewegungen kommen praktisch nicht vor.

Die Tremorfrequenz von zerebellärem Tremor bei Multipler Sklerose liegt unter 5 Hz. Die Behandlung des Tremors bei MS wird allgemein als schwierig angesehen. Es gibt keine etablierte Pharmakotherapie des zerebellären Tremors (Deuschl, G., Leitlinien für Diagnostik und Therapie in der Neurologie. Stuttgart, September 2012, Thieme Verlag).

Der essentielle Tremor, dessen Ursache bislang unbekannt ist, stellt die häufigste Form der Tremorerkrankungen dar. Die Tremorfrequenz liegt bei fünf bis sechs Hz. In 94% % der Fälle sind die Hände von diesem Tremor betroffen. Zahlreiche geprüfte Medikamente stehen zur Verfügung. Dennoch sprechen Patienten nicht unbedingt auf die Behandlung an, da der essentielle Tremor heterogen ist. Neben der begrenzten Wirksamkeit - die medikamentöse Therapie erweist sich im Mittel in rund 60 % der Fälle als wirksam - stellen oft Nebenwirkungen einen limitierenden Faktor dar.

Bestimmte Arten von Tremor können, wie vorstehend dargelegt, medikamentös behandelt werden, was jedoch mit Nebenwirkungen verbunden ist, oder chirurgisch durch gezieltes Zerstören bestimmter Hirnareale oder durch Implantation eines Hirnschrittmachers. Die chirurgische Behandlung ist - wie jede Operation - mit einem hohen Risiko verbunden und zudem nicht bei jedem Patienten erfolgreich.

Es ist ferner nicht möglich, einen Tremor durch Krankengymnastik zu verbessern oder zu beseitigen. Mit Entspannungsübungen wie autogenem Training, Yoga oder Meditation lassen sich Symptome nur kurzfristig verringern.

Als weitere Behandlungsmöglichkeit ist es aus der Praxis bekannt, den Tremor mittels Gewichtsmanschetten an den betroffenen Extremitäten zu verringern. Außerdem ist es möglich, beim Essen besonders schwere Bestecke zu verwenden. Die Wirksamkeit derartiger Ansätze ist jedoch nicht sehr zufriedenstellend. Zudem treten schnell Gewöhnungseffekte ein, was die Wirksamkeit weiter verringert.

Die bekannten Behandlungsmethoden von Tremor haben demzufolge je nach Ansatz das Problem, dass sie mit hohen Nebenwirkungen oder den mit operativen Eingriffen am Gehirn verbundenen hohen Gesundheitsrisiken verbunden sind oder nur kurzfristig oder unzureichend wirken.

Es ist somit eine Aufgabe der Erfindung, eine Vorrichtung und ein Verfahren bereitzustellen, mit denen Nachteile herkömmlicher Techniken vermieden werden können. Die Aufgabe der Erfindung ist es insbesondere, eine Vorrichtung bereitzustellen, die keine oder nur geringe Nebenwirkungen aufweist und mit der Tremor, insbesondere der Arme und Hände, deutlich verringert werden kann. Es ist eine weitere Aufgabe der Erfindung, von Tremor betroffenen Personen ein Hilfsmittel an die Hand zu geben, welches das Zittern eines Körperteils, vorzugsweise der Hand, verringert. Die erfindungsgemäße Vorrichtung soll nur zeitweise eingesetzt werden, und zwar genau dann, wenn eine Tätigkeit durchgeführt werden soll, welche eine ruhige Hand erfordert, beispielsweise während des Essens. Die erfindungsgemäße Vorrichtung soll dabei nicht die den Tremor verursachende Krankheit heilen, sondern lediglich das Symptom "Zittern" für eine gewisse Zeit reduzieren. Diese Aufgaben werden durch eine Vorrichtung gemäß Anspruch 1 gelöst. Vorteilhafte Ausführungsformen und Anwendungen der Erfindung sind Gegenstand der abhängigen Ansprüche und werden in der folgenden Beschreibung unter teilweiser Bezugnahme auf die Figuren näher erläutert.

Der Erfindung zugrunde liegt die allgemeine technische Lehre, eine am Körper tragbare, bei Bedarf anlegbare Vorrichtung bereitzustellen, die die vom Tremor erzeugte Zitterbewegung erkennt und diese aktiv kompensiert, indem wenigstens eine Masse, die ein Bestandteil der Vorrichtung ist, eine Gegenbewegung zu der Tremorbewegung des Körperteils ausführt.

Gemäß allgemeinen Gesichtspunkten der Erfindung wird eine am Körper tragbare Vorrichtung zum Reduzieren von Tremor eines Körperteils gemäß Anspruch 1 bereitgestellt. Die Vorrichtung umfasst ein Sensormittel zum Erzeugen eines Signals, das mit einem Tremor des Körperteils korreliert; einen Regler, der eingerichtet ist, in Abhängigkeit von dem Signal des Sensormittels eine Stellgröße zu bestimmen; und eine Massenverlagerungseinrichtung, umfassend mindestens ein Massenelement, die ausgeführt ist, das mindestens eine Massenelement in Abhängigkeit von der Stellgröße relativ zu dem Körperteil zu bewegen, derart, dass die Bewegung des mindestens einen Massenelements eine Gegenkraft auf den Körperteil ausübt, die einer durch Tremor verursachten Bewegung des Körperteils entgegenwirkt.

Die Vorrichtung, insbesondere das Sensormittel, der Regler und die Massenverlagerungseinrichtung bilden somit einen Regelkreis aus, mittels dessen durch entsprechende Verlagerung des mindestens einen Massenelements die Tremorbewegung aktiv kompensiert oder zumindest reduziert werden kann.

Ein besonderer Vorzug der Erfindung liegt darin, dass von Tremor betroffenen Personen ein Hilfsmittel bereitgestellt wird, welches das Zittern eines Körperteils verringert. Die erfindungsgemäße Vorrichtung kann dabei auch nur zeitweise eingesetzt werden, insbesondere dann, wenn eine Tätigkeit durchgeführt werden soll, welche z. B. eine ruhige Hand erfordert, beispielsweise während des Essens, so dass das Symptom "Zittern" für eine gewisse Zeit reduziert werden kann. Ein weiterer Vorteil ist, dass die erfindungsgemäße Vorrichtung dabei je nach Schwere der Erkrankung entweder dazu führen kann, dass der Betroffene seine Medikamentendosis verringern kann oder dass komplett auf eine medikamentöse Behandlung verzichtet werden kann, da der Tremor mit Hilfe der erfindungsgemäßen Vorrichtung während des Ausübens bestimmter Tätigkeiten ausreichend reduziert werden kann und im sonstigen Alltag das Zittern zwar unangenehm aber nicht behindernd ist. Ferner lässt sich die erfindungsgemäße Vorrichtung bei Tremorerkrankungen einsetzen, für die heute keine etablierte medikamentöse Therapie existiert (z. B. bei zerebellärem Tremor), oder bei Patienten, die auf die gängigen Therapien, wie z. B. medikamentöse Therapien, nicht oder nicht ausreichend ansprechen.

Für den von Tremor betroffenen Patienten stellt die erfindungsgemäße Vorrichtung somit eine neue alternative, nebenwirkungsfreie Behandlungsmethode für Tremorerkrankungen dar. Der direkte Nutzen für Patienten besteht darin, dass die erfindungsgemäße Vorrichtung das Zittern der Arme oder Hände unterbindet oder zumindest reduziert. Der indirekte Nutzen für Patienten besteht in der Vermeidung einer risikoreichen Implantation eines Hirnschrittmachers, einer Reduzierung bzw. Vermeidung einer Dauermedikation mit Medikamenten, die häufig starke Nebenwirkungen haben, und der einhergehenden Medikamentenkosten, die der Patient mittragen muss. Zudem wird die Lebensqualität stark erhöht (z. B. Alltagsbewältigung, Eigenständigkeit, Hobbies, soziale Integration).

Der direkte Nutzen für behandelnde Ärzte ist eine neue nebenwirkungsfreie Behandlungsalternative zur Therapie des Tremors als Ursache verschiedener Erkrankungsfälle. Abhängig von der Erkrankungsursache können so Risiken und Nebenwirkungen der anderen Therapien vermieden bzw. reduziert werden und Kosten eingespart werden.

Der direkte Nutzen für Krankenkassen und das Gesundheitssystem besteht im Einsparpotenzial des innovativen Medizinproduktes gegenüber der dauerhaften Medikamentenversorgung bzw. der teuren Hirnschrittmachertherapie. Regelmäßigen jährlichen Ausgaben für Medikamente von 1.000 bis 6.000 € bzw. 30.000 € für die Implantation eines Hirnschrittmachers steht die einmalige Ausgabe für eine erfindungsgemäße Vorrichtung gegenüber.

Weiterhin ist es denkbar, die erfindungsgemäße Vorrichtung im Falle eines verstärkten physiologischen Tremors einzusetzen. Auslöser des verstärkten physiologischen Tremors sind z. B. starke Aufregung, eine Vielzahl von Medikamenten und Drogenentzug (Deuschl, G., Leitlinien für Diagnostik und Therapie in der Neurologie, Stuttgart (September 2012), Thieme Verlag). Da die erfindungsgemäße Vorrichtung voraussichtlich keinerlei Nebenwirkungen haben wird, kann sie auch in diesen Fällen temporär zum Einsatz kommen, z. B. bei starker Aufregung. Ein mögliches Anwendungsszenario ist das Halten eines Vortrags. Dabei wird oft ein Laserpointer in der Hand gehalten, und der auf der Leinwand sichtbare Lichtpunkt macht das Zittern für alle Zuhörer offensichtlich. Das Verwenden der erfindungsgemäßen Vorrichtung am Arm des Vortragenden während der Dauer des Vortrags kann das Zittern verringern und somit die Nervosität des Vortragenden verbergen.

Der Körperteil ist vorzugsweise eine Gliedmaße bzw. Extremität, beispielsweise ein Arm, Oberarm, Unterarm oder ein Handgelenk. Die Vorrichtung umfasst ferner eine Befestigungseinrichtung, um die Vorrichtung ortsfest an einem Körperteil anzuordnen. Die Vorrichtung kann beispielsweise in Form einer Manschette oder stulpenförmig ausgestaltet sein, die sich am Körperteil, welches die Zitterbewegung ausführt, befestigen lässt.

Ferner kann die Vorrichtung Klettverschlüsse, ein Scharnier oder andere Befestigungsmittel aufweisen, um die Vorrichtung vorzugsweise kraftschlüssig an dem Körperteil zu befestigen. Besonders vorteilhaft ist das Vorsehen von aufblasbaren Fluidpolstern, um die Vorrichtung auf dem Körperteil anzuordnen, da mittels Fluidpolster ein besonders sicherer Sitz der Vorrichtung an dem Körperteil erreicht wird, was nachfolgend noch detaillierter beschrieben wird.

Die Befestigungseinrichtung kann als Halterung oder Aufnahme für die Massenverlagerungseinrichtung dienen oder zugleich als Teil der Massenverlagerungseinrichtung ausgeführt sein, was nachfolgend anhand der verschiedenen Ausführungsformen noch beschrieben wird.

Die Beschreibung ist hinsichtlich des konstruktiven Aufbaus der Vorrichtung nicht auf einen bestimmten Aufbau beschränkt, sondern die zuvor beschriebene allgemeine technische Lehre kann mittels unterschiedlicher Ausführungsvarianten umgesetzt werden. Nachfolgend werden einige vorteilhafte Ausführungsvarianten der Vorrichtung beschrieben. Die Erfindung ist in den Ansprüchen definiert

Die erfindungsgemäße Massenverlagerungseinrichtung umfasst zwei um eine gemeinsame Rotationsachse rotierbar gelagerte Massenelemente. Die beiden Massenelemente können somit im angelegten Zustand der Vorrichtung an einem Körperteil um den Körperteil rotieren,

d. h. sie sind kreisbogenförmig oder entlang einer Kreisbahn um den Körperteil bewegbar. Demgemäß ist der Regler ferner ausgeführt, eine Stellgröße zu bestimmen, die Rotationsbewegungen der beiden Massenelemente bewirkt, nämlich Rotationsbewegungen um den Körperteil, derart, dass eine aus den Rotationsbewegungen resultierende Trägheitskraft, nämlich Zentrifugalkraft, der durch Tremor verursachten Bewegung des Körperteils entgegenwirkt.

Ein besonderer Vorzug liegt darin,

dass eine Vorrichtung mit geringer Dicke bereitgestellt werden kann, da die Bewegungsbahn der beiden Massenelemente nahe am Körperteil entlang einer äußeren Umfangslinie verlaufen kann.

Bei einer vorteilhaften Variante der zwei

um eine gemeinsame Rotationsachse rotierbar gelagerten Massenelemente bewirkt die Stellgröße eine zyklische Hin- und Herbewegung der ersten der beiden Massenelemente entlang eines ersten Kreisbogens und eine korrespondierende zyklische Hin- und Herbewegung des zweiten Massenelements im gleichen Drehsinn oder im entgegengesetzten Drehsinn entlang eines zum ersten Kreisbogen gegenüberliegenden zweiten Kreisbogens. Die korrespondierende zyklische Hin- und Herbewegung des zweiten Massenelements erfolgt vorzugsweise synchron und in Phase oder gegenphasig zum ersten Massenelement.

Eine vorteilhafte Möglichkeit der Realisierung dieser Variante sieht vor, dass jedes der Massenelemente zwei bestrombare, miteinander bewegungsgekoppelte kreisbogenförmige Spulen aufweist, die relativ zu einem kreisbogenförmigen Tauchkern bewegbar geführt sind und sich im bestromten Zustand relativ zum Tauchkern bewegen, wobei eine zyklische Hin- und Herbewegung der Spulen durch ein abwechselndes Bestromen der Spulen erzeugbar ist. Der magnetisierbare Tauchkern ist starr zum Körperteil angeordnet, z. B. an einer das Körperteil umgreifenden Manschette. Die gegenüberliegenden Stirnbereiche greifen jeweils in den Spuleninnenraum einer der Spulen ein, d. h., der erste Stirnbereich taucht in den Innenraum der ersten Spule ein, der gegenüberliegende Stirnbereich in den Innenraum der zweiten Spule. Der Tauchkern jedes Massenelements kann hierbei einteilig, zweiteilig oder mehrteilig ausgeführt sein. Statt eines einteiligen Tauchkerns zwischen den beiden Spulen kann für jede Spule ein eigener Tauchkern vorgesehen sein, wobei die Tauchkerne dann unmittelbar oder mittelbar fest miteinander verbunden sind.

Gemäß dieser Variante dient somit die bestrombare Spule - auch Tauchspule genannt - eines Elektromagneten bzw. Hubmagneten als bewegliche Masse. Eine Massenverlagerungseinrichtung auf Basis derartiger Hubmagneten kann kompakt und robust ausgeführt werden.

Eine Weiterbildung dieser Variante sieht vor, dass jedes der Massenelemente eine die beiden Spulen umgebende kreisbogenförmige Kapsel umfasst, mit der die Spulen fest verbunden sind, wobei die Kapsel auf einer das Körperteil umgebenden Manschette oder Ringkörper, mit der oder dem der Tauchkörper fest verbunden ist, relativ zum Tauchkern bewegbar geführt ist. Die Spulen sind somit fest in eine gemeinsame Kapsel eingebettet, so dass sie über die Kapsel starr miteinander verbunden sind und in einem definierten Abstand voneinander angeordnet sind. Die Kapsel dient somit einerseits zur Beweglichen Lagerung der Spulen und kann durch ihr Eigengewicht ferner als zusätzliche bewegte Masse dienen.

Zur Reduzierung von Zitterbewegungen, die in verschiedenen Richtungen auftreten oder auch eine rotative Zitterbewegung aufweisen, sieht eine Weiterbildung der Erfindung vor, dass die zwei um die gemeinsame Rotationsachse rotierbar gelagerten Massenelemente im Innenraum eines ersten Ringkörpers oder einer ersten Teilmanschette vorgesehen sind und dass ein zweiter im wesentlichen baugleicher Ringkörper bzw. eine zweite Teilmanschette vorgesehen ist, in dem/der zwei weitere um die gemeinsame Rotationsachse rotierbar gelagerte Massenelemente vorgesehen sind, wobei der zweite Ringkörper gedreht, vorzugsweise um 90°, zum ersten Ringkörper angeordnet ist, so dass sich die Raumrichtung der von der Bewegung der Massenelemente des ersten Ringkörpers erzeugten Gegenkraft von derjenigen des zweiten Ringkörpers unterscheidet.

Bei einer weiteren vorteilhaften Variante der Ausführungsform mit zwei um eine gemeinsame Rotationsachse rotierbar gelagerten Massenelementen umfasst die Massenverlagerungseinrichtung zwei drehbar gelagerte und jeweils mit einem Antrieb versehene Ringkörper, an denen jeweils eines der Massenelemente ortsfest befestigt ist. Im angelegten Zustand der Vorrichtung umgreifen die Ringkörper somit den Körperteil und sind um den Körperteil drehbar gelagert. Ist die Vorrichtung ausgebildet, am Arm getragen zu werden, verläuft die gemeinsame Drehachse beispielsweise entlang der Längsachse des Arms.

Das Massenelement, das am Ringkörper befestigt ist, hat eine höhere Dichte als das Material des Ringkörpers und erzeugt somit eine Unwucht des Ringkörpers. Vorzugsweise sind die Massenelemente jeweils in den Ringkörper eingebracht, indem an wenigstens einer Stelle des Ringkörpers das leichte Ringkörpermaterial durch das schwerere Material des Massenelements ersetzt wird. Der Ringkörper kann aus einem starren leichten Material wie z. B. Plastik gebildet sein, die Massenelemente können z. B. aus Metall sein.

Gemäß einer Variante dieser Ausführungsform können die beiden Ringkörper auf einer Halterung mit einer zylinderförmigen Oberfläche drehbar gelagert sein. Zur Lagerung können Kugellager verwendet werden. Ferner kann die Massenverlagerungseinrichtung zwei Antriebe, z. B. zwei Motoren aufweisen, die zum Drehantrieb der Ringkörper jeweils mit einem der Ringkörper in Wirkverbindung stehen. Vorzugsweise sind die beiden Ringkörper nebeneinander, d. h. in Richtung der gemeinsamen Rotationsachse axial verschoben, angeordnet.

Gemäß einer weiteren Variante dieser Ausführungsform umfasst die erfindungsgemäße Vorrichtung ein Gehäuse, das zur Umgreifung des Körperteils ringförmig oder manschettenförmig ausgebildet ist, wobei an einer dem Körperteil zugewandten Außenfläche des Gehäuses ein aufpumpbares Fluidpolster angeordnet ist. Eine oder mehrere Aktoren zum Aufpumpen des Fluidpolsters können in dem Gehäuse angeordnet sein, so dass das Gehäuse durch Aufpumpen des Fluidpolsters ortsfest am Körperteil befestigbar ist. Gemäß dieser Variante sind die Ringkörper mittels Kugellager im Inneren des Gehäuses um den Körperteil drehbar gelagert und unabhängig voneinander antreibbar. Die zwei Motoren, die zum Drehantrieb der Ringkörper jeweils mit einem der Ringkörper in Verbindung stehen, sind vorzugsweise in das Gehäuse integriert.

Bei den vorgenannten konstruktiven Ausführungsvarianten bewirkt die vom Regler bestimmte Stellgröße eine Rotation der beiden Ringkörper mit den integrierten Massenelementen um das Körperelement. Aufgrund der Unwucht entstehen Zentrifugalkräfte. Mittels der Stellgröße wird die Rotation der beiden Ringkörper so geregelt, dass die resultierenden Zentrifugalkräfte der durch Tremor verursachten Bewegung des Körperteils entgegenwirken.

Anstatt Massenelemente, wie vorstehend beschrieben, starr auf drehbar angetriebenen Ringkörpern anzuordnen, so dass Zentrifugalkräfte bei der Drehbewegung der Ringkörper um den Körperteil erzeugt werden, kann die gleiche Wirkung auch dadurch erzielt werden, dass die Ringkörper starr zum Körperteil angeordnet sind und die Massenelemente auf der Oberfläche eines oder zweier verschiedener Ringkörper um den Körperteil herum verfahren werden.

Gemäß dieser alternativen Ausführungsvariante umfasst die Massenverlagerungseinrichtung zwei ortsfest zum Körperteil anordenbare Ringkörper. Die Ringkörper umgreifen jeweils den Körperteil, wobei jeweils eines der Massenelemente auf einem der beiden Ringkörper entlang eines Kreisbogens oder einer Umfangskreisbogenlinie verfahrbar gelagert ist. Alternativ kann die Massenverlagerungseinrichtung nur einen ortsfest zum Körperteil anordenbaren Ringkörper umfassen, auf dessen äußerer Mantelfläche die beiden Massenelemente axial versetzt zueinander verfahrbar gelagert sind, wobei die Verfahrbewegung der Massenelemente wiederum entlang eines Kreisbogens oder entlang einer Kreisbahn erfolgt. Gemäß dieser Ausführungsvarianten umfasst die Massenverlagerungseinrichtung einen Aktor zur Erzeugung der Verfahrbewegung der Massenelemente auf dem Ringkörper.

Es wird betont, dass die vorstehend beschriebenen Varianten auch dahingehend abgewandelt werden können, dass mehr als zwei Massenelemente vorgesehen sind, die auf starr angeordneten Ringkörpern verfahren werden oder die mit zwei oder mehr rotierbaren Ringkörpern rotiert werden können. Beispielsweise können vier Massenelemente vorgesehen sein, wobei entweder nur zwei der Massen oder alle vier Massen rotiert werden. Durch eine Erhöhung der Anzahl der bewegten Massen kann die Größe der erzeugten Gegenkraft angepasst werden.

Ferner ist zu erwähnen, dass gemäß der Ausführungsvarianten mit starr angeordneten Ringkörpern diese im Innern des zuvor genannten Gehäuses starr angeordnet sein können. Alternativ können die Ringkörper, auf denen die Massenelemente verfahren werden, selbst als Halterung für ein den Körperteil umgreifendes Fluidpolster und als Gehäuse für Pumpen, die Aktoren etc. dienen.

In einer zweiten Variante der Erfindung kann jedes der beiden Massenelemente zwei Teilmassen umfassen, die vorzugsweise das gleiche Gewicht aufweisen, die auf einer Umfangslinie des Ringkörpers des jeweiligen Massenelements angeordnet sind und deren Abstand zueinander variierbar ist.

Beispielsweise kann der Abstand der beiden Teilmassen zueinander über eine von einem Motor angetriebene Gewindestange variiert werden. Diese Ausführungsvariante bietet den Vorteil, dass dadurch die Unwucht der Rotationsbewegung des Massenelements variiert werden kann. Je näher die zwei Teilmassen zusammenliegen, desto größer ist die Amplitude der von einer Rotationsbewegung der Massen verursachten Gegenbewegung. Zur Verdeutlichung dieses Aspekts ist anzumerken, dass in dem Fall, wo sich die beiden Teilmassen diametral zum Körperteil gegenüberstehen würden, der Ring mit den beiden Teilmassen keine Unwucht besitzen würde, bzw. dass sich die Zentrifugalkräfte der beiden Teilmassen, falls diese das gleiche Gewicht haben, aufheben, so dass dem Zittern des Körperteils keine entsprechende Gegenbewegung entgegengesetzt werden kann. Sitzen die beiden Teilmassen jedoch dicht beisammen, so ergibt sich eine maximale Unwucht und so eine Gegenbewegung maximaler Amplitude bzw. Gegenkraft.

Für die vorstehend beschriebenen Varianten sind zur Reduzierung des Tremors verschiedene Regelvarianten möglich.

In einer ersten Variante ist der Regler eingerichtet, eine erste Stellgröße zu bestimmen, die jeweils eine Kreisbewegung der beiden Massenelemente mit vorzugsweise gleicher Winkelgeschwindigkeit, aber entgegengesetzter Drehrichtung bewirkt. Der Fall unterschiedlicher Winkelgeschwindigkeiten ist auch möglich. Insbesondere kann der Regler gemäß dieser Ausführungsvariante eingerichtet sein, die Stellgröße so zu bestimmen, dass eine Gerade durch die beiden gegenüberliegenden Stellen entlang der Kreisbewegung, an denen die beiden Massenelemente aneinander vorbeilaufen, parallel zu der zu reduzierenden Tremorbewegung ist.

Mit anderen Worten existieren aufgrund der gegenläufigen Bewegung der Massenelemente zwei gegenüberliegende Orte auf der kreisförmigen Umlaufbahn, die auf gegenüberliegenden Seiten des Körperteils liegen, an denen sich die beiden Massen "begegnen". Die Gerade durch diese beiden Punkte definiert die Stelle betragsmäßig maximaler Zentrifugalkraft entlang der Umlaufbahn, die von den Massenelementen erzeugt wird. Die Richtung der Zentrifugalkraft in den beiden Punkten ist entgegengesetzt.

Die Bewegung der Massenelemente wird somit so eingestellt, dass die Gerade parallel zu der Richtung der Zitterbewegung, die kompensiert wird, ist. Der Regler bestimmt die Stellgröße ferner so, dass die Winkelgeschwindigkeit, d. h. Rotationsfrequenz der Massenelemente gleich der Tremorfrequenz ist und dass die Richtung der Zentrifugalkraft jeweils entgegengesetzt zur Richtung der Tremorbewegung ist.

In einer zweiten Variante ist der Regler eingerichtet, eine zweite Stellgröße zu bestimmen, die eine zyklische Hin- und Herbewegung der beiden Massenelemente um einen Ausgangspunkt jeweils entlang eines Kreisbogens, vorzugsweise entlang eines Halbkreises, mit vorzugsweise gleicher Winkelgeschwindigkeit, aber entgegengesetzter Drehrichtung bewirkt. Der Fall unterschiedlicher Winkelgeschwindigkeiten ist auch möglich.

Die beiden Massenelemente bewegen sich hierbei synchron auf unterschiedlichen Seiten des Körperteils, d. h. die beiden Massenelemente durchlaufen gleichzeitig jeweils ihren Ausgangspunkt und erreichen gleichzeitig den oberen und unteren Umkehrpunkt ihrer Hin- und Herbewegung. In den oberen und unteren Punkten ist die Überlagerung der Trägheitskräfte der beiden Massenelemente maximal, an den Ausgangspunkten minimal.

Gemäß dieser Ausführungsvariante kann der Regler insbesondere eingerichtet sein, die Stellgröße so zu bestimmen, dass eine Gerade durch die beiden gegenüberliegenden Ausgangspunkte der Auslenkungen der beiden Massenelemente senkrecht zu der zu reduzierenden Tremorbewegung steht.

In einer dritten Variante ist der Regler eingerichtet, zur Reduzierung einer rotativen Zitterbewegung eine dritte Stellgröße zu bestimmen, die eine zyklische Hin- und Herbewegung der beiden Massenelemente mit vorzugsweiser gleicher Winkelgeschwindigkeit in die gleiche Drehrichtung bewirkt. Gemäß dieser Variante bewegen sich die beiden Massenelemente somit synchron nebeneinander auf der gleichen Seite des Körperteils. Der Fall unterschiedlicher Winkelgeschwindigkeiten ist auch möglich.

In der Ausführungsvariante, bei der jedes der beiden Massenelemente zwei Teilmassen umfasst, die auf einer Umfangslinie des Ringkörpers des jeweiligen Massenelements angeordnet sind und deren Abstand zueinander variierbar ist, kann der Regler eingerichtet sein, eine vierte Stellgröße zu bestimmen, die eine Änderung des Abstands zwischen den zwei Teilmassen bewirkt. Mit dieser vierten Stellgröße kann die Amplitude der Gegenkraft angepasst werden.

Es besteht auch die Möglichkeit, dass die Vorrichtung in verschiedenen Betriebsmodi betrieben werden kann, so dass die beiden Massenelemente wahlweise gemäß der ersten, der zweiten oder der dritten Stellgröße bewegt werden können. Die Stellgröße bewirkt somit die Stellung der Massenelemente relativ zueinander und/oder die Einstellung der Rotationsgeschwindigkeit der Massenelemente. Die vierte Stellgröße kann zusätzlich zu der ersten bis dritten Stellgröße bestimmt werden.

Je nachdem, ob ein drehbar gelagerter Ringkörper mit starr befestigten Massenelementen oder ein starrer Ringkörper mit auf dem Ringkörper verfahrbar angeordneten Massenelementen eingesetzt wird, bewirkt die Stellgröße somit eine Drehbewegung des Ringkörpers mit den starr eingefügten Massenelementen oder ein entsprechendes Verfahren der Massenelemente entlang des oder der Ringkörper.

Ein besonderer Vorteil der vorgenannten Varianten ist, dass durch entsprechendes Stellen der beiden Massenelemente relativ zueinander die Richtung, in der die erzeugten Gegenkräfte zur Kompensation bzw. Reduzierung von Zitterbewegungen entstehen, auf die Achse der Zitterbewegung des Körperteils eingestellt werden kann. Durch Anpassung der Rotationsgeschwindigkeit kann die Vorrichtung an patientenspezifische Zitterfrequenzen angepasst werden. Durch entsprechendes Stellen des Abstands zweier Teilmassen, bzw. der Amplitude der zyklischen Hin- und Herbewegung der Massenelemente, kann die Amplitude der der Zitterbewegung entgegenwirkenden Kraft eingestellt werden. Zusätzlich kann über den Aufbau aus mehr als zwei Ringelementen durch die Hinzunahme eines zweiten Ringpaars somit also die Hinzunahme eines weiteren Massepaars zur Unterdrückung der Zitterbewegung die resultierende Gesamtmasse verändert werden.

Gemäß einer weiteren nicht-beanspruchten Ausführungsvariante umfasst die Massenverlagerungseinrichtung ein Fluidpolster, auf dem das Massenelement gelagert ist, wobei das Fluidpolster mindestens eine erste und eine zweite Fluidkammer umfasst, die durch eine Pumpeneinrichtung fluidverbunden sind. Unter Fluid soll in diesem Zusammenhang eine Flüssigkeit oder ein Gas verstanden werden.

Gemäß dieser nicht-beanspruchten Ausführungsvariante ist die Massenverlagerungseinrichtung ferner ausgeführt, in Abhängigkeit der Stellgröße Fluid zwischen der mindestens einen ersten und mindestens einen zweiten Fluidkammer hin und her zu bewegen, derart, dass das auf dem Fluidpolster gelagerte Massenelement eine Hin- und Herbewegung ausführt.

Gemäß dieser nicht-beanspruchten Ausführungsvariante kann somit das Massenelement über Verlagerung von Fluid innerhalb des Fluidpolsters bewegt werden. Hierzu sind die mindestens zwei Kammern des Fluidpolsters vorzugsweise so anordenbar, dass die Bewegungsrichtung des Massenelements parallel zur Tremorrichtung ist. Dies kann beispielsweise auch einfach dadurch sichergestellt werden, dass die Vorrichtung um den Körperteil entsprechend gedreht wird, bis die Bewegungsrichtung des Massenelements parallel zur Zitterbewegung ist. Ferner wird vorzugsweise die Frequenz der Fluidverlagerung der Frequenz der Tremorfrequenz angepasst.

Bei einer vorteilhaften Variante dieser nicht-beanspruchten Ausgestaltungsform umfasst das Fluidpolster zwei Gruppen mit jeweils mindestens einer ersten und einer zweiten Fluidkammer, die so angeordnet sind, dass eine durch Fluidverlagerung innerhalb der ersten Gruppe erzeugte Hin- und Herbewegung des Massenelements entlang einer ersten Raumachse erfolgt und eine durch Fluidverlagerung innerhalb der zweiten Gruppe erzeugte Hin- und Herbewegung des Massenelements entlang einer zweiten Raumachse erfolgt, die von der ersten Raumachse verschieden ist. Besonders vorzugsweise steht die erste Raumachse senkrecht zur zweiten Raumachse. Bei einer weiteren besonders vorteilhaften Variante sind jeweils drei Gruppen von Fluidkammern mit jeweils mindestens zwei Fluidkammern in jeder Gruppe vorgesehen.

Gemäß einer weiteren Variante der vorgenannten nicht-beanspruchten Ausführungsvarianten, aufweisend das Fluidpolster zur Bewegung des Massenelements, kann das Massenelement als eine Manschette aus einem schweren Werkstoff, vorzugsweise Metall, ausgeführt sein, die auf dem Fluidpolster gelagert ist. Die Bezeichnung "schwerer" Werkstoff bezieht sich auf Werkstoffe mit verhältnismäßig hoher Dichte, insbesondere mit einer höheren Dichte als das Fluid, so dass die durch die Fluidverlagerung entstehenden Kräfte klein sind im Vergleich zu den Kräften, die durch die Bewegung der Manschette entstehen.

Es besteht jedoch auch die Möglichkeit, dass das Massenelement als Gel ausgeführt ist, das in Form eines Gelkissens auf dem Fluidpolster gelagert ist, wobei das Gel eine höhere Dichte als das Fluid des Fluidpolsters aufweist und somit als träge Masse dient. Beispielsweise kann das Fluidpolster als ein Luftkissen ausgebildet sein, so dass durch die unterschiedliche Befüllung der Luftkissen eine Massenänderung durch das Verdrängen des Gels erreicht wird.

Gemäß einer weiteren Variante der vorgenannten nicht-beanspruchten Ausführungsbeispiele kann die Vorrichtung ferner ein äußeres Fluidpolster aufweisen, das auf dem beweglichen Massenelement, beispielsweise der Manschette, aufliegt und dieses überdeckt. Dies hat den Vorteil, dass eine durch die Massenverlagerungseinrichtung erzeugte Bewegung des Massenelements zwar zu einer Verdrängung von Fluid in dem äußeren Fluidpolster führen kann, jedoch die Bewegung des Massenelements von außen nicht sichtbar ist. Demzufolge ist nicht sichtbar, dass der Tremorpatient eine sich bewegende Manschette trägt.

Gemäß einer weiteren vorteilhaften Variante der vorgenannten nicht-beanspruchten Ausgestaltungsformen, aufweisend das Fluidpolster zur Bewegung des Massenelements, umfasst die Vorrichtung zur Reduzierung von Tremor ein Gehäuse, dass zur Umgreifung des Körperteils ringförmig ausgebildet ist, und mindestens zwei Gruppen mit jeweils über die Pumpeneinrichtung gekoppelte Fluidkammern

Gemäß dieser Variante sind die ersten Fluidkammern jeder Gruppe an einer dem Körperteil zugewandten Außenfläche des Gehäuses angeordnet, und die zweiten Fluidkammern jeder Gruppe sind gegenüberliegend zu den jeweiligen ersten Fluidkammern im Inneren des Gehäuses angeordnet. Im angelegten Zustand des Gehäuses am Körperteil umgibt dieses somit ringförmig den Körperteil, wobei die ersten Fluidkammern vorzugsweise ebenfalls ringförmig um den Körperteil herum angeordnet sind und durch Aufpumpen der ersten Fluidkammern eine kraftschlüssige Verbindung zwischen der Vorrichtung und dem Körperteil erzeugen.

Gemäß einem weiteren Aspekt ist die Pumpeneinrichtung, die beispielsweise mehrere Pumpen für jede Gruppe von Fluidkammern umfassen kann, in der Gehäusewand integriert. Das Gehäuse kann beispielsweise aus Plastik gebildet sein. Die Gehäuseform kann beispielsweise hohlzylinderförmig, manschettenförmig oder stulpenförmig ausgeführt sein. Besonders vorteilhaft ist, wenn drei Gruppen mit jeweils zwei über jeweils eine Pumpe gekoppelten Fluidkammern vorgesehen sind.

Eine weitere konstruktive Möglichkeit der Realisierung der Massenverlagerungseinrichtung umfasst: eine erste Manschette, die ortsfest an dem Körperteil anbringbar ist, eine zweite Manschette aus einem schweren Werkstoff, vorzugsweise Metall, als Massenelement, mindestens eine Nockenwelle und einen Antrieb der mindestens einen Nockenwelle. Die zweite Manschette hat einen größeren Durchmesser als die erste Manschette und ist konzentrisch zu dieser angeordnet und mittels der mindestens einen Nockenwelle beweglich auf der ersten Manschette gelagert. Die äußere Manschette ist somit auf den Nocken bzw. den Vorsprüngen der Nockenwelle gelagert, so dass sich durch Drehen der Nocken der Abstand der äußeren Manschette zur inneren Manschette periodisch verändert. Vorzugsweise ist hierbei die Nockenwelle an einer der beiden Manschetten befestigt.

Gemäß dieser Ausführungsvariante erzeugt eine Rotation der Nockenwelle eine Relativbewegung der beiden Manschetten. Die Hin- und Herbewegung der äußeren Manschette kann über die Stellgröße so festgelegt werden, dass die Bewegung der schweren Manschette als träges Massenelement eine Kraft erzeugt, die der Tremorbewegung entgegenwirkt.

Gemäß einer vorteilhaften Variante der vorgenannten Ausführungsform umfasst die Vorrichtung zwei sich gegenphasig bewegende und auf gegenüberliegenden Seiten der ersten Manschette angeordnete Nockenwellen, so dass die Zitterbewegung besonders wirksam in einer Raumdimension ausgeglichen werden kann. Besonders vorteilhaft ist eine Ausführungsvariante, bei der die Vorrichtung zwei Paare von je zwei sich gegenphasig bewegenden und auf gegenüberliegenden Seiten der ersten Manschette und zwischen der ersten und der zweiten Manschette angeordneten Nockenwellen umfasst, wobei die Nockenwellen des ersten Paares um 90° versetzt zu den Nockenwellen des zweiten Paares angeordnet sind. Die Nockenwellen des ersten Paares sind somit um 90° in Umfangsrichtung des Körperteils versetzt zum ersten Paar angeordnet, so dass zwei Richtungskomponenten der Zitterbewegung ausgeglichen werden.

Es besteht jedoch auch die Möglichkeit, eine weitere nichtbeanspruchte Ausführungsvariante vorzusehen, bei der die Massenverlagerungseinrichtung einen auf dem Körperteil anordenbaren Vibrationsmotor umfasst, wobei das Massenelement als Unwucht des Vibrationsmotors ausgeführt ist. Derartige Vibrationsmotoren verwenden eine nicht rotationssymmetrische Masse, die auf der Motorachse sitzt und rotiert. Dabei ist mindestens ein Vibrationsmotor an der Manschette angebracht und kann die Zitterbewegung in einer Richtung kompensieren.

Vorzugsweise umfasst die Massenverlagerungseinrichtung dieser nicht-beanspruchten Ausführungsvariante zwei Vibrationsmotoren, die in zwei linear unabhängigen Richtungen vibrieren, um die Zitterbewegung in zwei linear unabhängige Richtungen kompensieren zu können. Die nicht rotationssymmetrische Masse kann ferner so ausgeführt sein, dass die Unsymmetrie veränderbar ist, um so verschieden große Vibrationsamplituden einstellen zu können. Ein Verändern der Unsymmetrie ist beispielsweise durch Verwenden einer Masse möglich, welche an einem Linearmotor befestigt ist. Mittels des Linearmotors kann der Abstand des Massenschwerpunkts von der Drehachse des ersten Motors verändert werden.

Gemäß einem weiteren Aspekt der Erfindung können der Regler und/oder das Sensormittel einen ersten Filter umfassen, der Frequenzen oberhalb eines oberen Grenzwerts herausfiltert. Beispielsweise kann der Filter so ausgelegt sein, dass er Grenzen oberhalb eines Grenzwerts von 20 Hz herausfiltert. Tremorfrequenzen liegen je nach Art des Tremors ca. im Bereich von 4 bis 15 Hz. Folglich stammen Zitterbewegungen höherer Frequenz nicht vorn Tremor und werden durch den ersten Filter herausgefiltert.

Schließlich besteht im Rahmen der Erfindung die Möglichkeit, dass der Regler oder das Sensormittel einen zweiten Filter umfassen, der Frequenzen unterhalb eines unteren Grenzwerts, beispielsweise unterhalb von 3 Hz, herausfiltert.

Damit kann sichergestellt werden, dass bewusst ausgeführte Bewegungen (beispielsweise Essbewegungen) durch das erfindungsgemäße Gerät nicht unterdrückt werden. Diese Bewegungen weisen niedrige Frequenzen auf.

Selbstverständlich können die Funktionen der beiden genannten Filter auch ein einem einzigen, sogenannten Bandpassfilter, vereint sein, welcher Frequenzen unterhalb eines unteren Grenzwertes und oberhalb eines oberen Grenzwertes herausfiltert.

Besonders vorteilhaft ist, wenn die Vorrichtung eine Einstellmöglichkeit vorsieht, den oberen Grenzwert und/oder den unteren Grenzwert festzulegen, um die Grenzwerte patientenspezifisch anpassen zu können.

Ferner kann die Vorrichtung eingerichtet sein, über den Regler, d.h. die Reglerelektronik, den oberen und unteren Grenzwert eigenständig aus den Sensorsignalen zu ermitteln und aus in dem Regler bereits vorhandenen Filtern die Filter mit den ermittelten Grenzwerten auszuwählen und im Weiteren zu verwenden.

Weiterhin können in der Literatur (siehe z. B. Kalyana C. Veluvolu and Wei Tech Ang, Estimation of Physiological Tremor from Accelerometers for Real-Time Applications, Sensors 2011, 11, 3020-3036; doi:10.3390/s110303020) bereits beschriebene Algorithmen eingesetzt werden, welche folgende Aufgaben lösen:

a) Trennen der Tremor bedingten Sensorsignale von störenden hochfrequenten Einflüssen, deren Frequenzen oberhalb der erwarteten Tremorfrequenzen liegen, wie z. B. Rauschen.
b) Trennen der Tremor bedingten Sensorsignale von störenden niederfrequenten Sensorsignalen, deren Frequenzen unterhalb der erwarteten Tremorfrequenzen liegen, wie z. B. der Einfluss der Erdbeschleunigung oder Signale, die von willentlich ausgeführten Handbewegungen resultieren.

Zum Lösen dieser Aufgabe sind beispielsweise die in der oben angegebenen Literaturstelle beschriebenen Algorithmen "Fourier Linear Combiner", "Weighted-frequency Fourier linear combiner", "Band limited multiple Fourier linear combiner" oder die Kombination des "Band limited multiple Fourier linear combiner" mit einem Kalman-Filter geeignet. Vor allem die Algorithmen "Weighted-frequency Fourier linear combiner" und "Band limited multiple Fourier linear combiner" können als adaptive Algorithmen ausgestaltet sein, welche eine zeitliche Änderung der Tremorfrequenz berücksichtigen. Weiterhin bieten diese Algorithmen den Vorteil, dass sie, im Gegensatz zu einem normalen, z. B. als Butterworth-Filter ausgeführten Bandpassfilter, nicht zu einer Phasenverschiebung der Sensorsignale führen. Dies ist im Hinblick auf eine zeitlich nicht verzögerte aktive Gegensteuerung der Tremorbewegung besonders vorteilhaft.

Es ist bekannt, dass als "Band limited multiple Fourier linear combiner" ausgeführte Bandpassfilter nur dann zufriedenstellende Resultate liefern, wenn das Frequenzspektrum des Eingangssignals keine nennenswerten Peaks außerhalb des durchzulassenden Frequenzbands enthält. Im Falle der erfindungsgemäßen Vorrichtung sind oberhalb der oberen Grenzfrequenz des Bandpassfilters vor allem auf ein Rauschen zurückzuführende Anteile im Sensorsignal zu erwarten. Diese haben im Frequenzspektrum keinen ausgeprägten Peak. Im Gegensatz dazu können aus willentlich ausgeführten Bewegungen resultierende oder dem Einfluss der Gravitationskraft geschuldete niederfrequente Anteile der Beschleunigungssensorsignale einen nennenswerten und unterhalb der unteren Grenzfrequenz des Bandpassfilters liegenden Peak im Frequenzspektrum aufweisen.

Daher ist es besonders vorteilhaft, wenn die erfindungsgemäße Vorrichtung einem als "Band limited multiple Fourier linear combiner" ausgeführten Bandpassfilter eine erste Filterung voranstellt, welche Signalanteile mit Frequenzen unterhalb der unteren Grenzfrequenz des Bandpassfilters aus den Sensorsignalen herausfiltern. Das Ausgangssignal dieser Vorfilterung soll gegenüber dem ursprünglichen Sensorsignal keine Phasenverschiebung aufweisen. Dies kann beispielsweise erreicht werden, indem die Beschleunigungssensorsignale einen Tiefpassfilter durchlaufen und anschließend das Ausgangssignal des Tiefpassfilters vom ursprünglichen Beschleunigungssensorsignal subtrahiert wird. Das nach der Subtraktion erhaltene Signal enthält keine niederfrequenten Anteile und ist gegenüber dem ursprünglichen Beschleunigungssensorsignal nicht phasenverschoben. Dieses Verfahren sowie eine mögliche anschließende Filterung mittels eines "Band limited multiple Fourier linear combiner" als Bandpassfilter sind beispielsweise in (W. T. Latt, U-X. Tan, K. C. Veluvolu, C. Y. Shee, and W. T. Ang, Physiological Tremor Sensing using only Accelerometers for Realtime Compensation, Proceedings of the 2008 IEEE International Conference on Robotics and Biomimetics Bangkok, Thailand, February 21 - 26, 2009) beschrieben.

Gemäß einem weiteren Aspekt wird das Sensormittel so an der Vorrichtung angeordnet, dass das Sensormittel beim Befestigen der Vorrichtung an dem Körperteil anliegt, beispielsweise entgegen dem Arm oder das Handgelenk gedrückt wird, um so die Beschleunigung des Körperteils detektieren zu können. Beispielsweise kann der Beschleunigungssensor fest mit der Manschette verbunden werden, um beim Anlegen der Manschette gegen den Arm oder das Handgelenk gedrückt zu werden. Bei einer Befestigung der Manschette am Arm kann außerdem das Sensormittel, z. B. mit der Manschette durch ein Kabel verbunden, auf der Hand befestigt sein. Alternativ kann das auf der Hand befestigte Sensormittel mit der Möglichkeit ausgestattet sein, die Sensordaten drahtlos an die Manschette zu übertragen, z. B. mittels Funk. Die erfindungsgemäße Vorrichtung bewegt dann den Arm aktiv so, dass die über das Handgelenk mit dem Arm verbundene Hand keine Zitterbewegung ausführt.

Vorzugsweise umfasst das Sensormittel einen Beschleunigungssensor. Aus der gemessenen Beschleunigung des Körperteils kann auf die Frequenz und Stärke des Tremors geschlossen werden. Weiter vorzugsweise ist das Sensormittel ausgeführt, Beschleunigungen des Körperteils in mehrere Raumrichtungen zu erfassen. Hierzu kann das Sensormittel mindestens zwei Sensoren umfassen, die so an der Vorrichtung angeordnet sind, dass sie versetzt zueinander, beispielsweise 90° versetzt zueinander, am Körperteil anordenbar sind. Alternativ kann das Sensormittel auch als Mehrachsensensor ausgeführt sein. Zur Erfassung von rotativen Tremorbewegungen kann das Sensormittel auch einen Drehratensensor umfassen.

Neben den vorgenannten Ausführungsvarianten, bei denen das mindestens eine Massenelement entlang eines Kreises oder einer Kreisbogenlinie bewegt wird oder bei denen ein Massenelement durch ein Fluidpolster periodisch bewegt wird, sind weitere andere konstruktive Ansätze möglich, um die allgemeine technische Lehre der Erfindung umzusetzen.

Beispielsweise kann die Massenverlagerungseinrichtung ausgeführt sein, das mindestens eine Massenelement auf einer mechanischen Blattfeder, an einem Gummi, an einer Saite, mittels einer Gewindestange oder in einem Kanal bzw. einer Bohrung beweglich zu lagern und durch einen Aktor in Abhängigkeit der Stellgröße zur Erzeugung einer Trägheitskraft anzuregen. Die Anregung des Massenelements kann mittels einer Schwingspule - auch Tauchspule genannt - oder mittels anderer auf der Kraftwirkung eines von einer stromdurchflossenen Spule erzeugten Magnetfelds beruhenden Prinzipien, elektrostatisch über einen Motor oder andere geeignete Mittel erfolgen. Vorzugsweise werden wiederum mehrere Massen verwendet, die sich in verschiedene Richtungen bewegen lassen. Alternativ ist es selbstverständlich auch möglich, eine Masse zu verwenden, die so gelagert ist, dass sie in verschiedene Richtungen, beispielsweise verschiedene Transaktionsrichtungen bewegbar gelagert ist. Zur Kompensation von Drehbewegungen ist es vorteilhaft, eine drehbar aufgehängte Masse vorzusehen.

Gemäß weiteren Aspekten kann der Regler als PID-Regler ausgeführt sein.

Ferner kann die Vorrichtung eine Energieversorgungseinheit aufweisen. Die Energieversorgungseinheit ist vorzugsweise eine Batterie oder ein Akkumulator. Beispielsweise kann das Gerät so ausgestaltet sein, dass sich der Akkumulator z. B. nachts drahtlos mittels einer induktiven Energieübertragung aufladen lässt. Alternativ kann eine Brennstoffzelle als Energieversorgungseinheit zum Einsatz kommen.

Ferner besteht im Rahmen der Erfindung die Möglichkeit, die Masse des mindestens einen Massenelements veränderbar auszugestalten, beispielsweise um die von der Vorrichtung erzeugbare Gegenkraft an unterschiedlich starken Tremor anpassen zu können. Hierzu kann beispielsweise die Aufnahme des Massenelements der Vorrichtung so ausgestaltet sein, dass das Massenelement austauschbar ist oder die Vorrichtung mit einer variierbaren Zahl von Massenelementen bestückbar ist.

Die Beschreibung betrifft ferner ein Regelverfahren zum Betrieb einer Vorrichtung. Das Verfahren umfasst die folgenden Schritte: Erfassen des Signals des Sensormittels als Führungsgröße; Bestimmen der Stellgröße in Abhängigkeit von der Führungsgröße, wobei die Stellgröße iterativ so bestimmt wird, (a) dass eine Bewegungsamplitude, ein Gewicht und/oder eine Bewegungsfrequenz des Massenelements zunehmen, falls das Signal des Sensormittels einer Zunahme der tremorbedingten Beschleunigung des Körperteils entspricht; und (b) dass eine Bewegungsamplitude, ein Gewicht und/oder eine Bewegungsfrequenz des Massenelements abnehmen, falls das Signal des Sensormittels einer Abnahme der tremorbedingten Beschleunigung des Körperteils entspricht; und (c) dass eine Bewegungsamplitude, ein Gewicht und/oder eine Bewegungsfrequenz des Massenelements im Wesentlichen unverändert bleiben, falls das Signal des Sensormittels einer zum Erliegen gekommenen Beschleunigung des Körperteils entspricht; und Bewegen des mindestens einen Massenelements durch die Massenverlagerungseinrichtung gemäß der aktuellen Stellgröße.

Vorzugsweise wird die Stellgröße so bestimmt, dass sich eine Phasenverschiebung zwischen dem Sensorsignal und dem Regelsignal von vorzugsweise 180° einstellt, so dass sich das mindestens eine Massenelement gegenläufig zur Tremorbewegung des Körperteils bewegt.

Weitere Einzelheiten und Vorteile werden im Folgenden unter Bezug auf die beigefügten Zeichnungen beschrieben. Es zeigen:

Figur 1          eine Prinzipdarstellung der Vorrichtung zur Reduzierung von Tremor in einer vorderen Querschnittsansicht gemäß einem Ausführungsbeispiel;

Figur 2          das Ausführungsbeispiel in einer seitlichen Querschnittsansicht;

Figur 3          die Rotationsbewegung der beiden Massenelemente des Ausführungsbeispiels;

Figuren 3A-3D    Prinzipdarstellungen eines weiteren Ausführungsbeispiels;

Figuren 3E-3F    Prinzipdarstellungen eines weiteren Ausführungsbeispiels;

Figur 4          eine Prinzipdarstellung eines weiteren Ausführungsbeispiels in einer vorderen Querschnittsansicht;

Figur 4a         eine Prinzipdarstellung eines weiteren Ausführungsbeispiels in einer vorderen Querschnittsansicht;

Figur 5          eine Prinzipdarstellung eines weiteren Ausführungsbeispiels in einer seitlichen Querschnittsansicht; und

Figur 6          ein Ablaufdiagramm des Verfahrens.

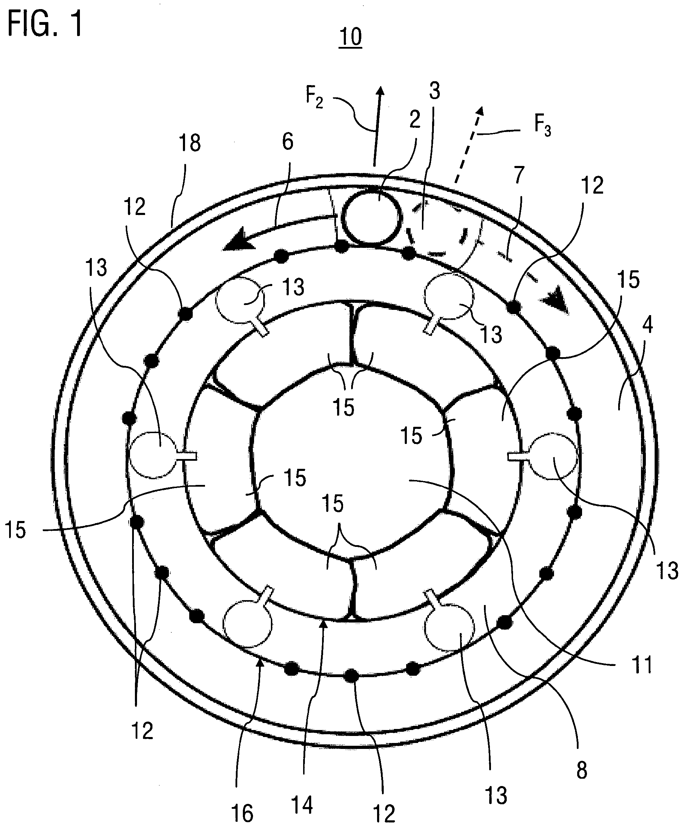

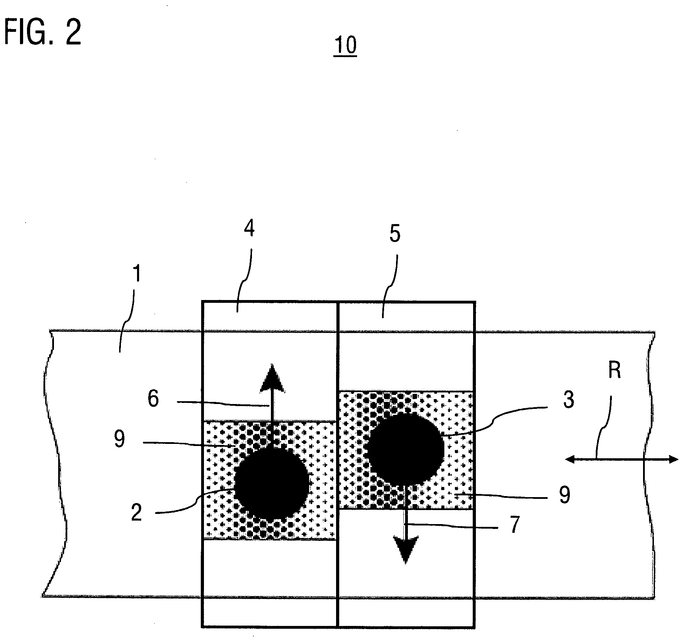

Figur 1 zeigt eine Prinzipdarstellung eines Ausführungsbeispiels der Erfindung in einer vorderen Querschnittsansicht, die einer Sicht in Richtung der Armachse entspricht. Figur 2 zeigt die an einem Arm 1 angeordnete Vorrichtung 10 in einer seitlichen Querschnittansicht auf den Arm 1.

Die Vorrichtung 10 ist manschettenförmig bzw. im Querschnitt ringförmig ausgebildet, so dass sie auf einen Armabschnitt 1 oder ein Handgelenk eines Tremorpatienten aufgezogen werden kann. Ein manschettenförmig bzw. ringförmig ausgebildetes Gehäuse 8 aus Kunststoff dient zur Aufnahme der Komponenten des Regelkreises, insbesondere zur Aufnahme der Komponenten der Massenverlagerungseinrichtung.

Der innere Bereich 11, der vom Gehäuses 8 ringförmig umschlossen wird, dient zur Aufnahme eines Armabschnitts und wird von sechs segmentförmig aufgebauten Luftkissen 15 begrenzt, die an einer dem Arm zugewandten inneren Mantelfläche 14 des Gehäuses 8 in Umfangsrichtung der Mantelfläche 14 nebeneinander angeordnet sind. Die Vorrichtung 10 wird über das Gehäuse 8 und insbesondere über die am Gehäuse angebrachten Luftkissen 15 am Körperteil befestigt.

Jedes Einzelluftkissen 15 kann separat über eine Pumpe 13 mit Luft gefüllt werden. Über ein Ventil, das an der Pumpe aufgesetzt ist (nicht gezeigt), und eine aus dem Gehäuse 8 herausführende Fluidleitung 22 kann die Pumpe 13 Luft von außen ansaugen bzw. ablassen und das Luftkissen 15 füllen bzw. leeren.

Die Pumpen 13 sowie der Regler, d.h. die gesamte Elektronik 60 zur Auswertung und Steuerung, sitzen in dem Gehäuse 8.

Das Plastikgehäuse 8 weist einen Innenraum 23 auf, der als Aufnahme für zwei entgegengesetzt laufende Ringkörper 4, 5 dient. Die Ringkörper 4, 5 sind im inneren Hohlraum 23 des Gehäuses 8 auf einer unteren Mantelfläche 16 des Hohlraums 23 über Kugellager 12 drehbar gelagert und können über zwei Motoren gegeneinander verdreht werden. Die Ringkörper sind beispielsweise aus Kunststoff gefertigt. Der Hohlraum wird in Radialrichtung des Körperteils nach außen hin durch eine äußere Gehäusewand 18 begrenzt, so dass die Bewegung der Ringkörper 4, 5 von außen nicht sichtbar ist.

Figur 2 illustriert in einer vereinfachten Darstellung die Anordnung der beiden Ringe 4 und 5, die einen Arm 1 umgreifen.

In jeden der beiden Ringen 4 und 5 ist jeweils eine Kugel 2 bzw. 3 aus Metall eingebracht. Beispielsweise kann in jedem Ring ein Teilsegment herausgeschnitten werden und durch ein passendes Segment 9 mit einer integrierten Metallkugel 2 bzw. 3 ersetzt werden.

Durch das Einbringen der Metallkugeln 2 bzw. 3 in die Ringe 4 bzw. 5 wird somit eine Unwucht erzeugt, so dass die Massenverteilung der Ringe 4, 5 mit eingebrachten Kugeln 2, 3 nicht mehr rotationssymmetrisch ist.

Wie in Figur 2 lediglich schematisch illustriert, sind die beiden Ringe 4, 5 in Axialrichtung des Armes 1 versetzt parallel zueinander angeordnet, so dass die beiden Ringe 4, 5 sich um die gleiche Rotationsachse R drehen. Bei einer Rotation der Ringe um den Arm rotieren die Kugeln 2, 3 somit um die Rotationsachse R und damit um den Arm 1. Die durch die Kugeln 2, 3 erzeugte Unwucht erzeugt bei der Rotation der Ringkörper 4, 5 Zentrifugalkräfte, die in Figur 1 für die Kugel 2 mit $F_2$ und für die Kugel 3 mit dem gestrichelten Pfeil $F_3$ gekennzeichnet sind.

Wie in den Figuren 1 und 2 mit den Pfeilen 6 und 7 dargestellt, rotieren die Ringkörper 4 und 5 in der gezeigten Betriebsart mit gleicher Winkelgeschwindigkeit, aber gegenläufig, so dass sich auch die Kugeln 2 und 3 mit gleicher Winkelgeschwindigkeit, aber entgegengesetzter Drehrichtung 6, 7 bewegen. Dies entspricht einer Regelung gemäß der vorgenannten ersten Stellgröße.

Stehen sich die beiden Kugeln 2, 3 umfangseitig diametral gegenüber, dann heben sich bei gleichen Massen der Kugeln 2, 3 die Zentrifugalkräfte $F_2$ und $F_3$ auf. An den Stellen, an denen die beiden Massenelemente 2, 3 aneinander vorbeilaufen, ist die resultierende Zentrifugalkraft $F_2 + F_3$ maximal.

Wird nun die Winkelgeschwindigkeit der umlaufenden Ringe 4, 5 an die Tremorfrequenz angepasst und werden die beiden Massenelemente 2, 3 so gestellt, dass die Gerade durch die beiden gegenüberliegenden Stellen entlang der Kreisbahn, an denen die beiden Massenelemente aneinander vorbeilaufen, parallel zu der zu reduzierenden Tremorbewegung ist, entsteht eine Kraft, die der Tremorbewegung entgegenwirkt.

Hierzu muss die Stellgröße so eingestellt werden, dass die resultierende Zentrifugalkraft um 180° phasenverschoben zu der Tremorbewegung ist, d. h. wenn sich der Körperteil durch die Tremorbewegung nach unten bewegt, müssen sich in dem Moment die beiden Massenelemente oben an der Vorrichtung 10 treffen, d. h. aneinander vorbeilaufen. Und umgekehrt, wenn sich der Körperteil 1 aufgrund des Tremors nach oben bewegt, müssen die Massenelemente so gestellt werden, dass sie sich in diesem Moment an der unteren Stelle des Körperteils an der Vorrichtung treffen.

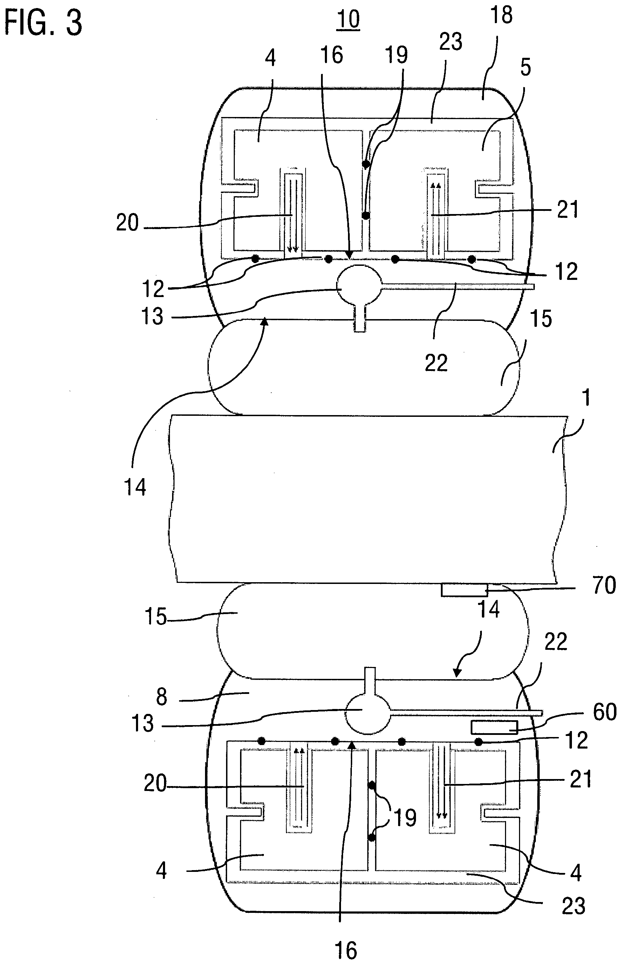

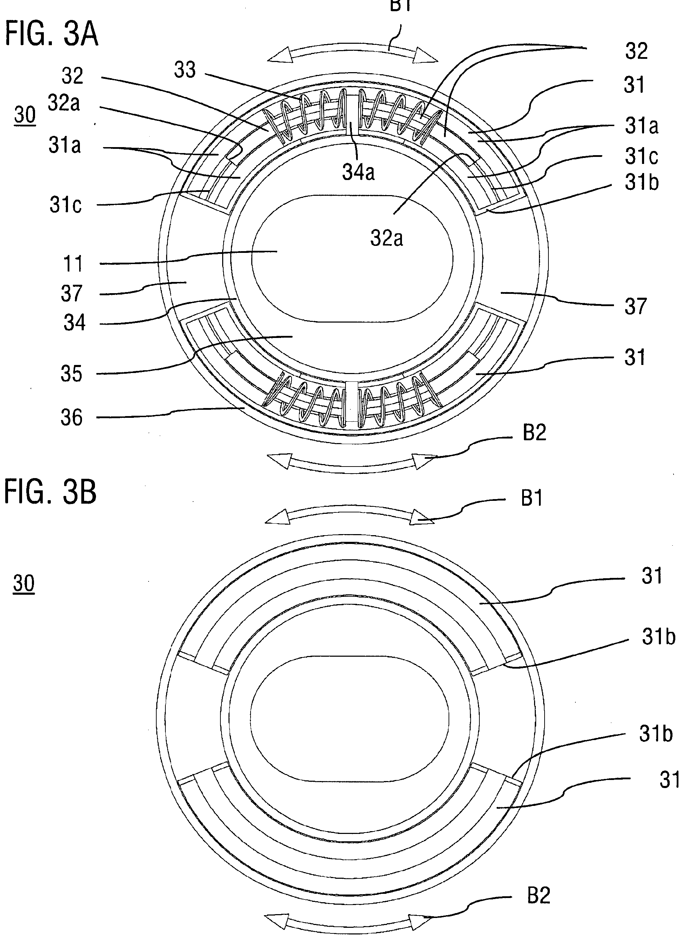

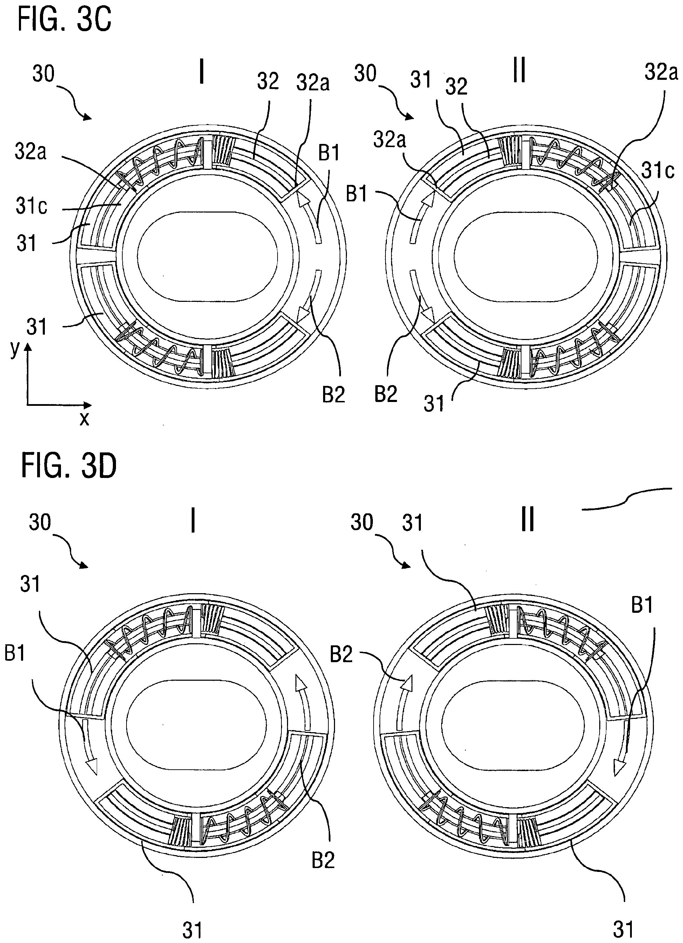

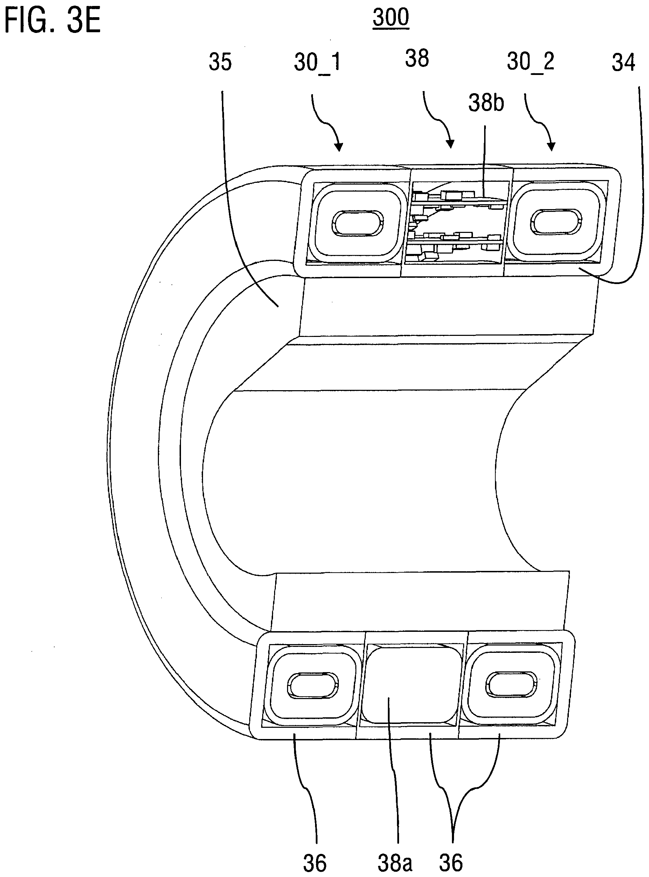

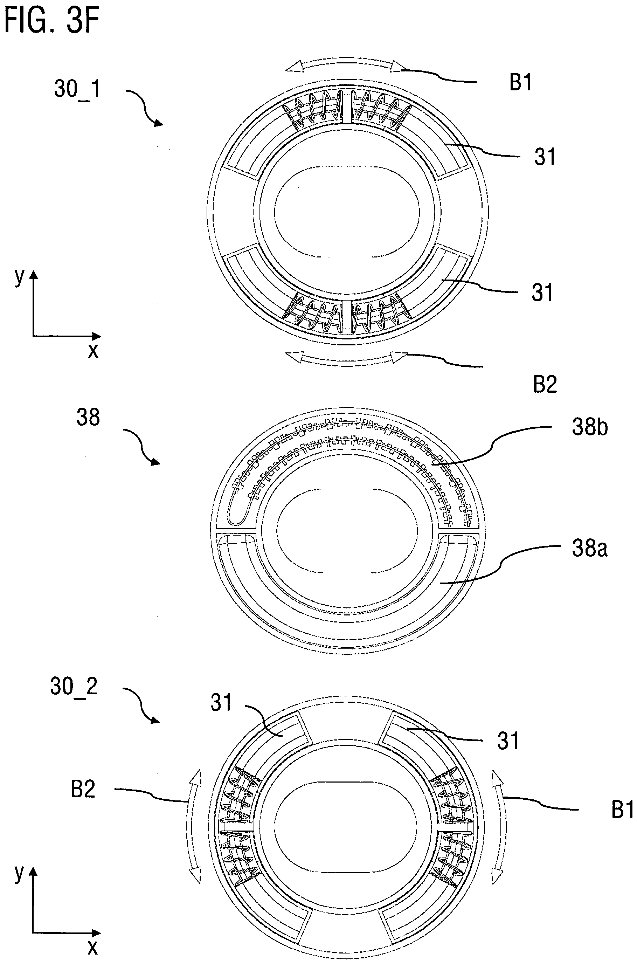

Figur 3 zeigt weiterhin, dass die beiden Ringkörper 4, 5 jeweils auf einem Laufrad 20, 21 gelagert sind. Jedes der Laufräder 20, 21 steht über eine Welle mit einem Motor in Wirkverbindung, so dass der Motor über die Welle das jeweilige Laufrad antreiben kann. Der Motor ist ebenfalls in dem Gehäuse 8 angeordnet und in Figur 3 nicht dargestellt. Das Laufrad 20 erzeugt die Drehbewegung des Rings 4 und das Laufrad 21 entsprechend die Drehbewegung des Rings 5. Zwischen den Ringen 4, 5 sind weitere Kugellager 19 vorgesehen, um eine relative Verdrehung der beiden Ringe zueinander zu unterstützen.

Ferner ist ein Beschleunigungssensor 70 an einer Innenseite eines Luftpolsters 15 so angeordnet, dass beim Anlegen der Vorrichtung 10 der Beschleunigungssensor 70 in Kontakt mit der Oberfläche des Körperteils kommt und gegen diese gedrückt wird. Der Sensor ist ausgebildet, die von der Zitterbewegung erzeugte Beschleunigung des Körperteils zu erfassen, und gibt das Sensorsignal an einen Regler 60 weiter, der eingangsseitig das Sensorsignal empfängt.

Der Regler 60 ist als PID-Regler ausgebildet und nutzt das Sensorsignal zur Regelung der Drehbewegung der Ringe 4, 5. Die Elektronik des Reglers 60 ist ferner mit einem Filter ausgestattet, welcher Frequenzen oberhalb eines Grenzwerts von 20 Hz herausfiltert, da bekanntermaßen höhere Frequenzen als 20 Hz nicht von einem Tremor resultieren. Ferner ist der Regler mit einem weiteren unteren Filter ausgestattet, der eingerichtet ist, Frequenzen unterhalb eines Grenzwerts von beispielsweise 3 Hz herauszufiltern, um sicherzustellen, dass bewusst ausgeführte Bewegungen des Patienten, z. B. Essbewegungen, nicht durch das erfindungsgemäße Gerät unterdrückt werden.

Die Motoren empfangen die vom Regler berechnete Stellgröße. In Abhängigkeit von der Stellgröße treibt jeder der Motoren denjenigen Ringkörper 4 bzw. 5 an, mit dem er in Wirkverbindung steht.

Es wurde vorstehend bereits erwähnt, dass je nach Betriebsmodus der Vorrichtung 10 der Regler verschiedene Stellgrößen bestimmen kann. Gemäß einem ersten Betriebsmodus ist der Regler eingerichtet, eine erste Stellgröße zu bestimmen, die jeweils eine Kreisbewegung der beiden Massenelemente mit gleicher Winkelgeschwindigkeit, aber entgegengesetzter Drehrichtung bewirkt.

Es besteht im Rahmen der Erfindung jedoch auch die Möglichkeit, dass der Regler eingerichtet ist, eine zweite Stellgröße zu bestimmen, die ein zyklisches Hin- und Herbewegen der beiden Massenelemente 2, 3 jeweils entlang eines Kreisbogens, vorzugsweise entlang eines Halbkreises, mit gleicher Winkelgeschwindigkeit, aber entgegengesetzter Drehrichtung bewirkt. Hier ist der Regler so eingerichtet, über die Stellgröße die Massenelemente 2, 3 so zu stellen, dass eine Gerade durch die beiden gegenüberliegenden Ausgangspunkte der Auslenkungen der beiden Massenelemente senkrecht zu der zu reduzierenden Tremorbewegung steht.

Gemäß dieser Variante werden somit die beiden Massenelemente, ähnlich der Unruhe einer mechanischen Uhr, zyklisch vor- und zurückbewegt. In der Ruheposition, d.h. dem Mittelpunkt der zyklischen Bewegung, stehen sich die Massen der beiden Ringe somit diametral gegenüber. Je nach Stärke bzw. Amplitude der Tremorbewegung werden die Massen aus der Ruheposition maximal +/- 90° zyklisch verfahren. Je größer die Amplitude der zyklischen Bewegung ist, desto größer ist die der Zitterbewegung entgegengesetzte Gegenbewegung. Die Richtung der Zitterbewegung, welche durch diese Gegenbewegung kompensiert werden kann, steht senkrecht auf der Verbindungslinie der Ruheposition der beiden Massen.

Mit der Vorrichtung können jedoch auch rotative Zitterbewegungen reduziert bzw. kompensiert werden. In diesem Fall ist der Regler eingerichtet, eine dritte Stellgröße zu bestimmen, die eine zyklische Hin- und Herbewegung der beiden Massenelemente 2, 3 mit gleicher Winkelgeschwindigkeit in die gleiche Drehrichtung bewirkt. In diesem Fall bewegen sich die beiden Massenelemente 2, 3 bzw. die Ringe 4, 5 somit im Gleichlauf synchron zueinander.

Weitere bevorzugte Ausführungsformen 30, 300, bei der die Massenelemente, ähnlich der Unruhe einer mechanischen Uhr, zyklisch vor- und zurückbewegt werden, sind in den Ansichten der Figuren 3A bis 3F illustriert. Gleiche oder funktional äquivalente Elemente sind in den Figuren mit denselben Bezugszeichen bezeichnet. Gemäß diesen Ausführungsformen erfolgt die Bewegung der Massen mittels einer elektromagnetischen Kraft, hervorgerufen durch eine stromdurchflossene Spule 31a. Dieses Prinzip ist von sogenannten Hubmagneten bekannt.

Die Schnittansicht der Figur 3A zeigt den Aufbau einer Ausführungsform 30 zur Reduzierung von Tremor eines Arms oder Handgelenks. Der innere Hohlraum 11 wird von einer weichen Anpassschicht 35 umgeben, die sich an den Arm oder das Handgelenk (nicht dargestellt) anlegt. Die Anpassschicht 35 ist an der dem Arm oder dem Handgelenk zugewandten Seite einer Manschette 34 mit ringförmigem Querschnitt befestigt. Auf der gegenüberliegenden Seite der Manschette 34 befindet sich ein Hohlraum 37, der von einer Außenverkleidung 36 verschlossen ist. Die Außenverkleidung ist fest mit der Manschette 34 verbunden.

In dem Hohlraum 37 sind zwei Massenelemente 31 beweglich gelagert, so dass diese eine Hin- und Herbewegung entlang eines Kreisbogens ausführen können.

Jedes der beiden Massenelemente 31 ist aus zwei bestrombaren, kreisbogenförmigen elektrischen Spulen 31a eines Elektromagneten gebildet sowie aus einer gemeinsamen Kapsel 31b, in die die beiden Spulen 31a fest eingebettet sind, so dass sie über die Kapsel 31b starr miteinander verbunden und in definiertem Abstand voneinander angeordnet sind. Mit dem Bezugszeichen 31 ist somit die Einheit aus den beiden Spulen 31a und der Kapsel 31b bezeichnet. In Figur 3A ist ein Schnitt durch die Kapsel 31b gezeigt, so dass die Komponenten im Innern der Kapsel sichtbar sind. In der Ansicht der Figur 3B sind die beiden Kapseln 31b im vollständig verschlossenen Zustand gezeigt, so dass von den beiden Massenelementen 31 jeweils nur die äußere Kapsel 31b sichtbar ist.

Die Spulen 31a erstrecken sich jeweils von einem Längsende der Kapsel 31b bis zu einer Feder 33, die an einem Halteabschnitt 34a befestigt ist und ein Anschlagen der Spule 31a am Halteabschnitt 34a verhindert.

Jede der Spulen 31a ist eine Spule eines als Hubmagneten ausgeführten Elektromagneten. Wie in Figur 3A ferner gezeigt ist, umfasst der Elektromagnet einen kreisbogenförmigen Tauchkern 32 in Form eines Eisenkerns, der über den Halteabschnitt 34a starr an der Manschette 34 und somit starr zum Körperteil angeordnet ist. Der magnetisierbare Tauchkern 32 dient als Tauchkern der beiden Spulen 31a, die an gegenüberliegenden Seite des Tauchkerns 32 angeordnet sind. Der Tauchkern 32 kann als gemeinsamer Tauchkern der beiden Spulen ausgeführt sein oder als zwei separate, mittelbar oder unmittelbar fest miteinander verbundene Tauchkerne ausgeführt sein. Die gegenüberliegenden Stirnbereiche 32a des Tauchkerns 32 greifen jeweils in den Spuleninnenraum 31c einer der Spulen 31a ein. Vorliegend ist somit der Tauchkern 32 starr angeordnet, während die Spulen 31a bewegbar zum Tauchkern 32 geführt sind.

Ein elektrisch erzeugtes Magnetfeld, das durch die Bestromung einer Spule 31a erzeugt wird, erzeugt eine Magnetkraft, aufgrund der sich die Spule 31a relativ zum Tauchkern 32 bewegt. Wird z. B. in Figur 3A die linke obere Spule 31a bestromt, erzeugt die resultierende Magnetkraft eine Bewegung des Massenelements 31 im Uhrzeigersinn (dargestellt als Zustand II in Figur 3C).

Für eine Rückbewegung entgegen dem Uhrzeigersinn bedarf es eines zweiten Elektromagneten in Form der rechten oberen bestrombaren Spule 31a. Wird diese Spule bestromt, während die andere ausgeschaltet ist, bewegt sich das Massenelement entgegen dem Uhrzeigersinn (dargestellt als Zustand I in Figur 3C).

Eine zyklische Hin- und Herbewegung der Spulen 31a bzw. der Massenelemente 31 ist durch ein abwechselndes Bestromen der Spulen 31a jedes Massenelements 31 erzeugbar.

Die beiden Spulen 31 der Hubmagnete bilden so gemeinsam mit der Kapsel 31b, in welche sie eingebettet sind, eine Einheit. Diese Einheit stellt eine der beweglichen Massen der erfindungsgemäßen Vorrichtung dar und kann relativ zu den Eisenkernen 32 entlang eines gewissen Teilsegments einer Kreisbahn bewegt werden, was durch den Pfeil B1 illustriert ist. Da jeder Eisenkern 32 über die Manschette 34 starr zum Arm angeordnet ist, erfolgt also die Bewegung der Masse entlang einem Teilsegment einer Kreisbahn um den Arm. Durch zyklisches An- und Abschalten der beiden starr miteinander verbundenen Hubmagnete lässt sich die beschriebene Masse zyklisch im / gegen den Uhrzeigersinn auf einem Teilsegment dieser Kreisbahn bewegen. Diese Bewegung ähnelt der Bewegung der Unruh eines Uhrwerks.

In Fig. 3C sind die beiden Endpositionen I, II der zyklischen Bewegung um die in Figur 3A und Figur 3B gezeigte Ruhelage dargestellt. Die obere Masse 31 und die untere Masse 31 haben unterschiedlichen Drehsinn. In der Endposition I ist die Masse 31 gegen den Uhrzeigersinn (Richtung B1) in die Endposition verfahren, in der der Tauchkern 32 vollständig in die jeweils rechte Spule 31a eingetaucht ist und in der die rechte Stirnseite 32a des Tauchkerns 32 sich am rechten Kapselende der Kapsel befindet. Die linke Stirnseite 32a des Tauchkerns 32 befindet sich dagegen am Eingang des Innenraums 32c der linken Spule. In der Endposition II sind die linken Spulen der Massenelemente 31 im Uhrzeigersinn soweit gegen den Tauchkern 32 verfahren, dass die linke Stirnseite 32a des Tauchkerns jeweils vollständig in den Spuleninnenraum 32c der linken Spule eingetaucht ist und sich am linken Kapselende befindet.

Diese Hin- und Herbewegung der Massen 31 ist geeignet, um Translationsbewegungen der Hand entlang der x-Achse zu kompensieren.

In Figur 3D ist der Fall dargestellt, dass sich die beiden Massen 31 mit gleichem Drehsinn bewegen, illustriert durch die Pfeile B1, B2. Dargestellt sind die beiden Endpositionen I und II der zyklischen Bewegung um die in Fig. 3A gezeigte Ruhelage. Diese Bewegung der Massen ist geeignet, Rotationsbewegungen der Hand um die Armachse zu kompensieren.

Figur 3E zeigt eine perspektivische Schnittansicht eines weiteren Ausführungsbeispiels 300. Je nach spezifischem Zittermuster des Patienten kann die Vorrichtung bzw. Gesamtmanschette 300 aus zwei oder drei hintereinander liegenden Teil-Manschetten 30_1, 38 und 30_2 aufgebaut sein. Dabei enthält die in der Mitte liegende Teilmanschette 38 keine beweglichen Massen, sondern eine Batterie 38a und die Elektronik 38b des Reglers. Die beiden äußeren Teilmanschetten 30_1 und 30_2 sind wie die in Figur 3A gezeigte Vorrichtung 30 aufgebaut und enthalten Aktoren zum Kompensieren von Zitterbewegungen entlang verschiedener Richtungen sowie von rotativen Zitterbewegungen. Falls ein Patient lediglich in einer Richtung zittert, so reicht es aus, zwei Teil-Manschetten 30_1 und 38 zu verwenden. Armseitig befindet sich wiederum eine weiche Anpassschicht, die an der dem Arm zugwandten Seite der Manschette 34 angeordnet ist.

Figur 3F zeigt den Aufbau und die Anordnung der drei Teilmanschetten 30_1, 38 und 30_2 aus Figur 3E in einer Schnittansicht und im auseinandergebauten Zustand.

Die erste Teilmanschette 30_1 umfasst wiederum zwei auf einem Kreisbogen hin- und herbewegbare Massenelemente 31, die aus zwei in eine gemeinsame Kapsel eingebettete bestrombare Spulen und der Kapsel gebildet werden, wie vorstehend in Zusammenhang mit Figur 3A beschrieben. Die erste Teilmanschette 30_1 ist so am Arm oder Handgelenk befestigt, dass mit ihr Zitterbewegungen in x-Richtung (bei einer Bewegung der beiden Massenelemente im entgegengesetzten Drehsinn) sowie rotative Zitterbewegungen um die Armachse (bei einer Bewegung der beiden Massenelemente im gleichen Drehsinn) kompensiert oder reduziert werden können.

Die zweite äußere Teil-Manschette 30_2 ist im Wesentlichen baugleich zu ersten Teil-Manschette 30_1 aufgebaut, jedoch im Vergleich zu ersten Teilmanschette 30_1 um 90° gedreht am Arm oder Handgelenk befestigt, so dass mit den hin- und herbewegbaren Massenelementen 31 Zitterbewegungen in y-Richtung (bei einer Bewegung der beiden Massenelemente im entgegengesetzten Drehsinn) sowie rotative Zitterbewegungen um die Armachse (bei einer Bewegung der beiden Massenelemente im gleichen Drehsinn) kompensiert oder reduziert werden können.

In der Mitte zwischen den beiden äußeren Teilmanschette 30_1 und 30_2 befindet sich die mittlere Teilmanschette 38, in der eine elektrische Leiterplatte 38b, die mit elektronischen Bauteilen bestückt ist, und eine Batterie 38a untergebracht sind. Alternativ könnten Batterie und Elektronik (je nach deren Baugröße) in der Anpassschicht 35 untergebracht sein. In diesem Fall kann auf die mittlere Teilmanschette 38 verzichtet werden.

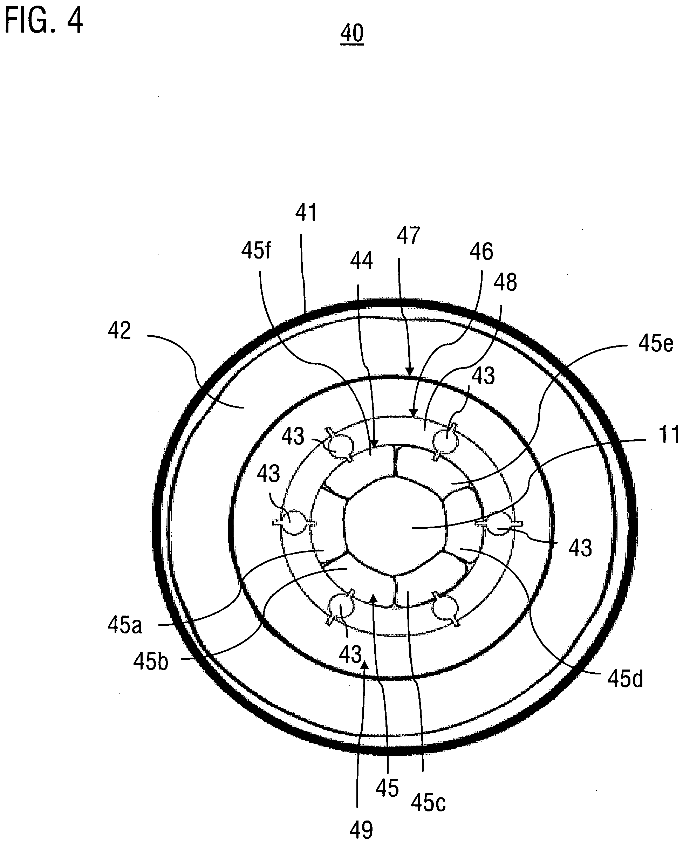

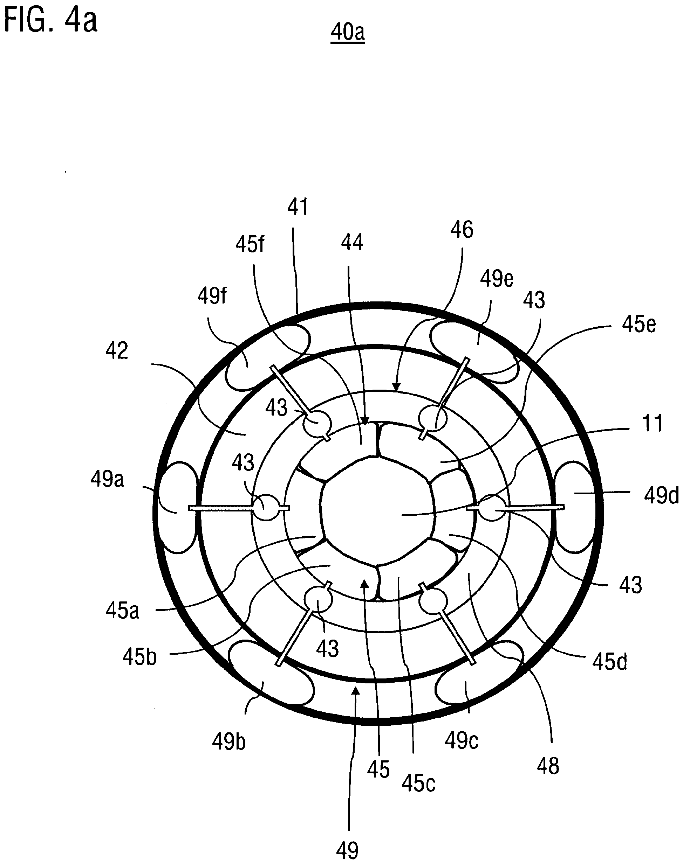

Figur 4 illustriert schematisch ein weiteres Ausführungsbeispiel 40 einer Vorrichtung zum Reduzieren von Tremor. Figur 4 zeigt wiederum eine Querschnittansicht in Richtung der Längsachse eines Arms, um den die Manschette 40 gelegt werden kann.

Eine Besonderheit dieses Ausführungsbeispiels liegt darin, dass eine schwere äußere Manschette 42 als Massenelement zur Erzeugung der Gegenkraft dient.

Die Vorrichtung umfasst wiederum ein manschetten- bzw. hohl-zylinderförmiges Plastikgehäuse 48. An der inneren Mantelfläche 44 des Gehäuses 48, d. h. der dem Körper zugewandten Innenseite des Gehäuses 48, die eine innere Manschette zur Umgreifung eines Armabschnitts ausbildet, sind wiederum sechs segmentförmig aufgebaute aufblasbare Luftkissen 45a bis 45f angeordnet.

Auf der gegenüberliegenden Seite 46 des Gehäuses 48 ist ein zweites Luftkissenpolster 49 angeordnet, das jedoch nicht unterteilt ist und als gemeinsames Fluidreservoir fungiert. Auf der äußeren Umfangsfläche 47 des zweiten Luftkissenpolsters 49 ist die schwere äußere Manschette 42 als Massenelement gelagert.

Hierbei ist jede Luftkammer des inneren Luftpolsters über eine Pumpe 43 und ein Ventil mit dem äußeren Luftpolster verbunden. Folglich führt ein Entleeren des inneren Luftkissens 45a und ein gleichzeitiges Füllen des gegenüberliegenden inneren Luftkissens 45d dazu, dass sich der Arm relativ zur zweiten schweren Manschette 42 bewegt. Dadurch wird die Tremorbewegung kompensiert. Entsprechendes gilt für die Paare von Luftkissenkammern 45b und 45e sowie 45c und 45f. Das äußere Luftkissenpolster 49, welchem die eine innere Luftkissenkammer Luft entnimmt, während ihm die gegenüberliegende innere Luftkissenkammer Luft zuführt, wird effektiv weder befüllt noch entleert.

Der Regler steuert die unterschiedlichen Kammern der Vorrichtung so an, dass die äußere Manschette 42 eine Auf- und Abbewegung entlang einer Raumrichtung durchführt, die parallel oder möglichst parallel zur Tremorbewegung ist. Da das Ausführungsbeispiel 40 mehrere Gruppen von Kammern hat, können auch Zitterbewegungen beispielsweise in zwei senkrecht aufeinander stehenden Raumachsen, die beide auch senkrecht auf der Unterachse des Arms stehen, kompensiert werden.

Figur 4a illustriert schematisch eine weitere Ausgestaltungsform.

Die Vorrichtung 40a umfasst wiederum ein manschetten- bzw. hohl-zylinderförmiges Plastikgehäuse 48. An der inneren Mantelfläche 44 des Gehäuses 48, d. h. der dem Körper zugewandten Innenseite des Gehäuses 48, die eine innere Manschette zur Umgreifung eines Armabschnitts ausbildet, sind wiederum sechs segmentförmig aufgebaute aufblasbare Luftkissen 45a bis 45f angeordnet.

Die schwere Manschette 42 ist in diesem Ausführungsbeispiel auf der äußeren Mantelfläche 46 des Gehäuses 48 gelagert. Auf der Außenseite der schweren Manschette 42 ist ein zweites Luftkissenpolster 49 angeordnet, das ebenfalls sechs Luftkammern 49a bis 49f aufweist. Hierbei ist jeweils eine Luftkammer des äußeren Luftpolsters über eine Pumpe 43 und ein Ventil mit einer Kammer des inneren Luftpolsters verbunden. Folglich führt ein Entleeren des inneren Luftkissens 45a, was zur Folge hat, dass die innere Manschette dichter am Arm liegt, zu einem Aufblasen des entsprechenden äußeren Luftkissens 49a. Entsprechendes gilt für die Paare von Luftkissenkammern 45b und 49b bis 45f und 49f.

Das äußere Luftpolster 49 ist von einer Gehäusewand 41 umgeben, so dass die Bewegung der Manschette 42 von außen nicht sichtbar ist. Insbesondere ist wegen des Verwendens von Paaren von Luftkammern 45a und 49a usw., bei denen sich jeweils eine Kammer zwischen Arm und schwerer Manschette 42 und die zweite Kammer zwischen schwerer Manschette 42 und Gehäusewand 41 befindet, wobei jeweils eine Kammer des Paares befüllt und gleichzeitig die andere entleert wird, von außen keine Bewegung zu sehen, obwohl sich im inneren der Manschette der Arm relativ zur schweren Manschette 42 bewegt.

Zum Anlegen der beiden Manschetten bzw. des Gehäuses 48 können diese mit einem Scharnier versehen sein.

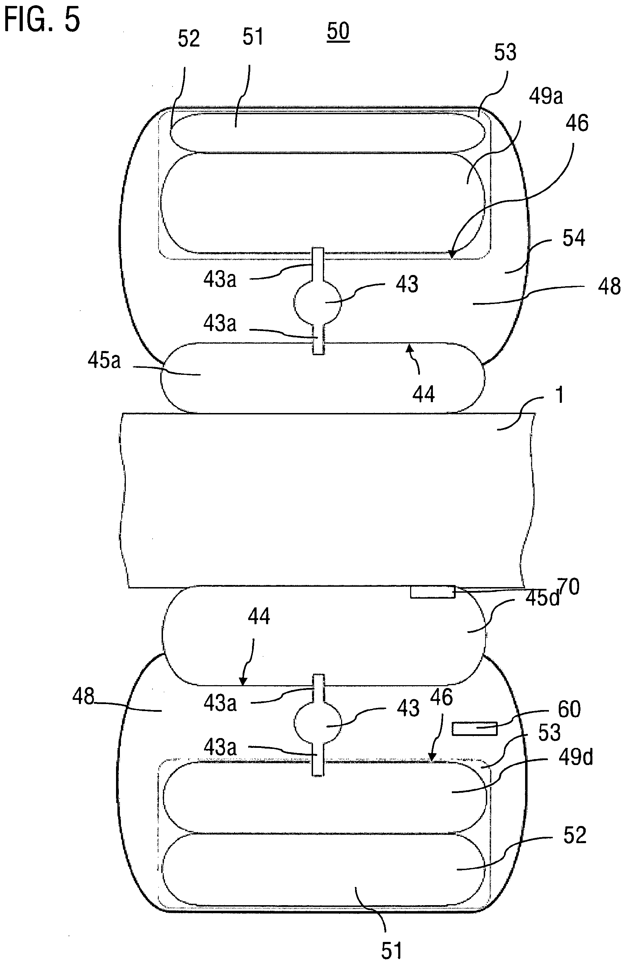

Figur 5 illustriert schematisch eine weitere Ausführungsvariante in einer seitlichen Querschnittsansicht. Die in Figur 5 gezeigte Vorrichtung 50 zeigt eine weitere Abwandlung des Ausführungsbeispiels der Figur 4, so dass zur Vermeidung von Wiederholungen auf die vorstehende Beschreibung verwiesen wird, wobei für entsprechende Einzelheiten dieselben Bezugszeichen verwendet werden.

Die Vorrichtung umfasst wiederum ein manschetten- bzw. hohl-zylinderförmiges Kunststoffgehäuse 48. An der inneren Mantelfläche 44 des Plastikgehäuses 48, d. h. der dem Körper zugewandten Innenseite des Gehäuses 48, die eine innere Manschette zur Umgreifung eines Armabschnitts ausbildet, sind wiederum sechs segmentförmig aufgebaute aufblasbare Luftkissen 45a bis 45f angeordnet, wobei in der Ansicht der Figur 5 nur das Luftkissen 45a und das untere Luftkissen 45d sichtbar sind. Das innere Luftpolster 45 ist wiederum über Pumpen 43 und entsprechende Luftkanäle 43a mit einem zweiten Luftkissenpolster 49 gekoppelt, das, im Gegensatz zum Beispiel von Figur 4, ebenfalls sechs Luftkammern 49a bis 49f aufweist. Hierbei ist jeweils eine Luftkammer des äußeren Luftpolsters über eine Pumpe 43 und ein Ventil mit einer Kammer des inneren Luftpolsters verbunden. Folglich führt ein Entleeren des inneren Luftkissens 45a, was zur Folge hat, dass die innere Manschette dichter am Arm liegt, zu einem Aufblasen des entsprechenden äußeren Luftkissens 49a. Entsprechendes gilt für die Paare von Luftkissenkammern 45b und 49b bis 45f und 49f.

In Figur 5 sind nur die Kammern 49a und 49d sichtbar.

Eine Besonderheit dieses Ausführungsbeispiels besteht darin, dass im Unterschied zu dem Ausführungsbeispiel der Figur 4 statt einer Manschette aus Metall nun ein Gel 51 als träge Masse in Form eines Gelkissens 52 auf dem zweiten Fluidpolster 49 bestehend aus den Fluidkammern 49a bis 49e gelagert ist. Das Gel hat eine höhere Dichte als das Fluid des Fluidpolsters.

Figur 5 zeigt eine Situation, in der Fluid aus dem Kissen 45a über die Pumpe 43 in das gegenüberliegend angeordnete Fluidkissen 49a gepumpt wird, wodurch Gel 51 aus dem Gelkissen 52 verdrängt wird. An der gegenüberliegenden Stelle des Arms 1 ist das Fluidkissen 45d jedoch noch voll befüllt, so dass kein Gel 51 aus dem unteren Bereich des Gelkissens 52 verdrängt wird. Durch abwechselndes Befüllen der Fluidkissen 45a und 45d kann somit eine entsprechende Hin- und Herverlagerung des Gels 51 von oben nach unten und umgekehrt erreicht werden.

Das entsprechende Regelverfahren erfolgt analog zu dem vorher beschriebenen Regelverfahren, wobei der Regler 60 in Abhängigkeit des erfassten Signals des Beschleunigungssensors 70 die als Aktor dienenden Pumpen 43 über die ermittelte Stellgröße so regelt, dass die entsprechende Verlagerung des Gels gegenphasig zu einer Zitterbewegung des Arms 1 erfolgt und somit eine der Tremorbewegung entgegengesetzte Kraft erzeugt wird.

Zur Verdeutlichung des Funktionsprinzips und zur Vereinfachung der Darstellung sind konstruktive Details wie beispielsweise Leitungen vom Sensor 70 zum Regler 60 oder Leitungen vom Regler 60 zu den Pumpen 43 in den Figuren 1 bis 5 nicht dargestellt. Ebenfalls nicht dargestellt ist eine Batterie, die ebenfalls in das Gehäuse 48 integriert ist, die zur Energieversorgung des Reglers der Sensorik und der Aktoren dient. Die Vorrichtung kann beispielsweise so ausgestaltet sein, dass sich der Akku nachts drahtlos wiederaufladen lässt, z. B. durch eine induktive Energieübertragung. In der Praxis wird dann die Manschette nachts auf eine Ladestation gelegt und Energie induktiv vom Ladegerät zur Spule übertragen.

Es besteht auch die Möglichkeit, dass mehrere Manschetten an einem Arm, z. B. am Oberarm und am Unterarm oder am Unterarm und am Handgelenk getragen werden können. In einer weiteren Variante und gemäß einer besonders einfachen Ausgestaltung ist es ebenfalls möglich, dass das Gerät keine aktive Regelung vornimmt. Stattdessen wird die Tremor-Schwingung lediglich gedämpft. Dies kann beispielsweise realisiert sein, indem die Manschette wassergefüllte Kammern enthält. Bei einer Bewegung des Arms bewegt sich der Arm in Richtung der Manschette, welche eine träge Masse darstellt, und verdrängt das Wasser aus der zwischen Arm und Manschette liegenden Kammer. Die Höhe der Dämpfung kann durch den fluidischen Widerstand eingestellt werden, den die Flüssigkeit beim Fließen von einer Kammer zur anderen überwinden muss. Diese hängt vor allem von der Länge und dem Durchmesser des dabei durchflossenen Kanals sowie von der Viskosität der Flüssigkeit ab. Weiterhin besteht die Möglichkeit, die Varianten der aktiven Regelung und der Dämpfung miteinander zu kombinieren.

Ferner ist zu erwähnen, dass es auch möglich ist, die Vorrichtung an einem Körperteil, beispielsweise Arm, zu befestigen und mittels eines über dem Befestigungsort hinausragenden Aktors die Zitterbewegung eines anderen Körperteils, z. B einer Hand, zu kompensieren.

Ferner ist es möglich, die Manschette an einem Körperteil, beispielsweise Arm, zu befestigen und das Sensormittel, z. B. mit der Manschette durch ein Kabel verbunden, an einem anderen Körperteil, z. B. Hand, zu befestigen. Die erfindungsgemäße Vorrichtung bewegt dann den Arm aktiv so, dass die über das Handgelenk mit dem Arm verbundene Hand keine Zitterbewegung ausführt. Bei dieser Ausführungsform kann eine von 180° abweichende Phasenverschiebung zwischen Sensorsignal und Aktorbewegung vorteilhaft sein.

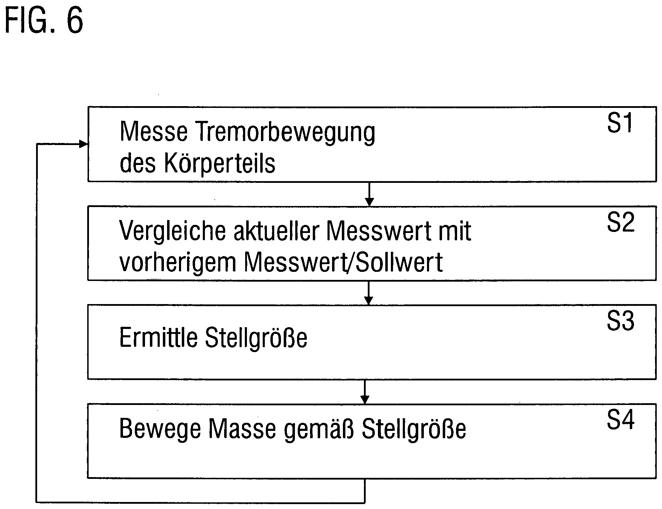

Ein Beispiel für ein Regelverfahren zum Betrieb der Vorrichtung ist in Figur 6 für das Ausführungsbeispiels der Figur 4 beschrieben.

Der Regler kann beispielsweise Software-technisch mittels eines Mikrocontrollers oder mittels einer Analogschaltung umgesetzt werden.

In Schritt S1 misst der Beschleunigungssensor des Sensormittels die Beschleunigung des Körperteils, die ein Maß für den Tremor ist. Als Führungsgröße des Regelkreises dient somit das Ausgangssignal des Beschleunigungssensors am Körperteil, nachdem niederfrequente Signale und Signale über einem oberen Grenzwert herausgefiltert wurden, wie zuvor beschrieben. Um das Zittern zu unterdrücken, muss das Ausgabesignal bzw. das Amplitudensignal des Beschleunigungssensors am Arm möglichst gering werden. Im Idealfall wird es auf null reduziert.

Hierzu wird das gemessene Signal über entsprechende Signalleitungen an den Regler übertragen. Dieser vergleicht in Schritt S2 den aktuellen Messwert mit dem vorherigen Messwert und dem vorgegebenen Sollwert und ermittelt daraufhin in Schritt S3 die resultierende Stellgröße.

Zeigt der Beschleunigungssensor am Körperteil einen hohen Ausschlag, wird die Stellgröße so festgelegt, dass die Manschettenamplitude und/oder das Manschettengewicht erhöht werden.

Letzteres, d. h. die Veränderung des Manschettengewichts, ist möglich, indem die Vorrichtungen gemäß den Figuren 1 bis 3 z. B. vier und nicht zwei umlaufende oder sich zyklisch bewegende Massenelemente (Manschettengewichte) aufweisen. Wahlweise können dann vier oder auch nur zwei Manschettengewichte verwendet werden.

Bei einer weiteren Variante der Ausführung einer Vorrichtung gemäß den Figuren 4 und 5 kann die Manschette aus einem hintereinander geschalteten dualen Zweikammersystem aufgebaut werden, welches zwei Kammern aufgebaut nach Figur 4 oder 5 enthält. Ist die Masse eines Gelkissens bzw. Masserings ausreichend, wird nur eine Kammer in Betrieb genommen. Bei einer notwendigen größeren Masse wird die zweite Kammer gleichphasig dazugeschaltet.

Ist dagegen die Beschleunigung am Arm rückläufig, erfolgt nur noch eine geringfügige Erhöhung der Manschettenamplitude. Falls die Beschleunigung am Arm zum Erliegen gekommen ist, werden alle zuvor über die Stellgröße eingestellten Parameter der Vorrichtung beibehalten.

In Schritt S4 wird dann über die Massenverlagerungseinrichtung die Masse gemäß der ermittelten Stellgröße bewegt. Anschließend fährt das Regelungsverfahren fort mit dem Schritt S1. Die Regelung wird somit iterativ durchgeführt, um festzustellen, ob die Parameteränderung in Schritt S3 und S4 positiv war, d. h. ob das Zittern zurückging oder ob die Parameteränderung negativ war, d. h. das Zittern wurde wieder stärker.

Vorzugsweise wird die Regelung so durchgeführt, dass das Signal des Beschleunigungssensors mit bestimmter Verstärkung und fest vorgegebener Phasenverschiebung, vorzugsweise einer Phasenverschiebung von 180°, zum Regeln des Aktors bzw. der Massenverlagerungseinrichtung verwendet wird. Der Verstärkungsfaktor wird so gewählt dass das Signal des Beschleunigungssensors minimiert wird. Die Frequenz des Aktors entspricht so automatisch immer der gemessenen Frequenz des Beschleunigungssensors. Vorzugsweise wird der Verstärkungsfaktor beim Einschalten der Regelung zunächst kontinuierlich einen vorgegebenen Wertebereich durchlaufen. Dabei wird der Wert ermittelt, welcher zu einem Minimum der Sensorwerte führt. Mit diesem Verstärkungsfaktor wird dann im weiteren Betrieb der Manschette die Regelung durchgeführt.

Als über die Stellgröße regelbare Parameter der Regelschleife können die folgenden Parameter der Vorrichtung eingesetzt werden: das Gewicht der Massenelemente, die Frequenz und die Amplitude der Bewegung des Massenelements und die Phasenverschiebung zwischen Sensorsignal und Regelsignal.

Obwohl die Vorrichtung unter Bezugnahme auf bestimmte Ausführungsbeispiele beschrieben worden ist, ist es für einen Fachmann ersichtlich, das verschiedene Änderungen ausgeführt werden können und Äquivalente als Ersatz verwendet werden können. Zusätzlich können viele Modifikationen ausgeführt werden, um eine bestimmte Situation oder ein bestimmtes Material an die Lehre anzupassen, ohne den zugehörigen Bereich zu verlassen.

Es wurde vorstehend bereits erwähnt, dass die Massenverlagerungseinrichtung bzw. der Aktor zum Kompensieren der Zitterbewegung des Körperteils konstruktiv auf verschiedenste Weise ausgeführt werden kann. Insbesondere wurde erwähnt, dass eine schwere Manschette als träge Masse verwendet werden kann, die durch Luft- oder Flüssigpolster bewegt werden kann oder alternativ beispielsweise auch durch die Lagerung der schweren Manschette auf den Nocken einer Nockenwelle, so dass das Drehen der Nockenwelle eine Auf- und Abbewegung der schweren Manschette bewirkt.

Weitere Ausführungsvarianten betreffen eine Massenverlagerungseinrichtung, die einen auf den Körper anordenbaren Vibrationsmotor umfasst, wobei das Massenelement als Unwucht des Vibrationsmotors ausgeführt ist. Weitere mögliche Ausführungsformen können gebildet werden durch das Anordnen einer Masse an einer mechanischen Blattfeder, die mit einem Befestigungsmittel am Körperteil befestigt wird. Die Anregung kann dann durch eine Schwingspule, elektrostatisch oder durch einen Motor erfolgen. Statt mit einer Blattfeder kann die Masse auch an einem Gummi, an einer Saite oder in einem Kanal bzw. einer Bohrung geführt sein und dort verschiebbar gelagert sein.

Nachfolgend werden experimentelle Ergebnisse erläutert, die zeigen, wie mit einer erfindungsgemäßen Vorrichtung die Tremorbewegung eines Körperteils reduziert werden kann.

Zur Bestimmung der zu erzeugenden und auf das vom Tremor betroffene Körperteil wirkenden Gegenkräfte werden folgende Überlegungen und Annahmen getroffen:

In diesem Beispiel ist die erfindungsgemäße Vorrichtung als am Handgelenk tragbare Manschette ausgestaltet, welche die Tremorbewegung des Unterarms reduziert, so dass mittels der an den Unterarm anschließenden Hand wieder Tätigkeiten durchgeführt werden können, welche eine ruhige Hand erfordern. Bei diesem Beispiel wird von einer ca. 70 kg schweren Person ausgegangen. Hand und Unterarm machen zusammen rund 3% des Körpergewichts aus und haben bei einer 70 kg schweren Person daher eine Masse von rund 2,1 kg. Es wird davon ausgegangen, dass der am Ellbogengelenk aufgehängte Unterarm mitsamt der Hand die Tremorbewegung ausführt. Da der Unterarm am Ellbogengelenk befestigt ist, muss die am Handgelenk getragene Manschette nicht den gesamten Unterarm mit voller Tremoramplitude bewegen, sondern nur das der Hand zugewandte leichtere Ende des Unterarms inkl. der Hand. Daher ist das effektiv zu berücksichtigende Gewicht des bewegten Arms, dessen Tremorbewegung durch die am Handgelenk befestigte Armmanschette zu reduzieren ist, etwas geringer als das tatsächliche Gesamtgewicht. Die folgende Abschätzung wird unter der Annahme eines effektiven Armgewichts von 1 kg durchgeführt.

Der Arm führt eine als sinusförmig angenommene Bewegung durch. Gerade bei den Erkrankungen Parkinsonsyndrom und essentieller Tremor stimmt die Annahme einer sinusförmigen Tremorbewegung der Hand sehr gut mit der Praxis überein.

Die Bewegung des Handgelenks kann mathematisch beschrieben werden durch folgende Gleichungen:

$$(\text{Formel 1}): \quad s = A \sin(2\pi f t)$$

$$(\text{Formel 2}): \quad a = -A \, (2\pi f)^2 \sin(2\pi f t)$$

$$(\text{Formel 3}): \quad F = m \, a,$$

wobei

s: Bewegungsverlauf (Ort) des Handgelenks,
t: Zeit,
A: Tremoramplitude am Ort des Handgelenks,
f: Tremorfrequenz,
a: aufgrund des Tremors auftretende Beschleunigung am Ort des Handgelenks,
m: effektiv zu bewegende Masse des Arms und
F: Kraft des schwingenden Arms, welcher die erfindungsgemäße Vorrichtung eine Gegenkraft entgegenbringen soll.

Durch Bewegen der bewegbaren Masse (Gewichte) der erfindungsgemäßen Vorrichtung mit der Frequenz der Tremorbewegung des Arms, jedoch mit einer dazu um 180° verschobenen Phasenlage, kann die Tremorbewegung des Arms reduziert werden.

Der Einfachheit halber wird davon ausgegangen, dass die Masse der in der Armmanschette zur Kompensation der Tremorbewegung eingesetzten Gewichte ebenfalls 1 kg beträgt und daher der Masse des Arms entspricht. Weiterhin gehen wir davon aus, dass die Tremorbewegung des Arms dann effizient reduziert werden kann, wenn die beweglichen Gewichte der Manschette bzgl. Frequenz und Amplitude den gleichen (sinusförmigen) Verlauf haben wie die Zitterbewegung der Hand. Lediglich die Phase muss sich von der Phase der sinusförmigen Handbewegung unterscheiden.

Die Frequenzen und Amplituden von Tremorbewegungen der Hand bei verschiedenen Erkrankungen sind bekannt und in der Literatur beschrieben. Bevorzugt soll die erfindungsgemäße Vorrichtung zur Reduzierung des Handtremors bei Patienten mit Parkinsonsyndrom oder mit essentiellem Tremor eingesetzt werden. Daher sind im Folgenden die bei diesen Erkrankungen auftretenden typischen Schwingungsamplituden und -frequenzen eines Handtremors beschrieben. Die angegebenen Daten wurden jeweils in Studien gewonnen, bei denen die Hand des Tremorpatienten mit einem Gewicht beschwert war. Diese Situation kommt dem Tragen der erfindungsgemäßen Vorrichtung sehr nahe:

Essentieller Tremor

In [Elble et al, Factors Influencing the Amplitude and Frequency of Essential Tremor, Movement Disorders Vol. 9, No. 6, 1994, pp. 589-596] wurde bei 59 Patienten mit essentiellem Tremor ein Gewicht von 300 g an der Hand befestigt und der Handtremor mittels eines Beschleunigungssensors ermittelt.

Im Mittel (Mittelung über alle untersuchten Patienten) ergaben sich eine Tremorfrequenz von 6,14 Hz und eine mittlere (Mittelung über alle untersuchten Patienten) Beschleunigungsamplitude in vertikaler Richtung von 1,96 m/s$^2$.

Aus den Werten für Frequenz f und Beschleunigungsamplitude $A(2\pi f)^2$ (siehe Gleichung 2) lässt sich die Amplitude der vertikalen Handauslenkung berechnen zu 1,3 mm.

Tremor bei Parkinson

In [RP Meshack and KE Norman, A randomized controlled trial of the effects of weights on amplitude and frequency of postural hand tremor in people with Parkinson's disease, Clinical Rehabilitation 2002; 16: 481-492] wurde bei 16 Patienten mit Parkinson eine Gewichtsmanschette mit einem Gewicht von 470 g an der Hand befestigt und die Amplitude des Handtremors gemessen. Je nach Messmethode ergaben sich im Mittel Tremoramplituden von 0,53 mm, 1,39 mm und 0,36 mm. Die mittlere Tremorfrequenz lag bei 5,56 Hz. Die durchgeführten Experimente haben ergeben, dass das bloße Beschweren der Handgelenke mit passiven Gewichten keinen signifikanten Einfluss auf die Tremorfrequenz und -amplitude hatte.

Die nachfolgenden experimentellen Ergebnisse wurden mit einer Ausführungsform erzielt, die im Wesentlich einen Aufbau wie die in Figur 3A gezeigte Vorrichtung hat, mit dem Unterschied, dass vier Hubmagnete verwendet wurden, die als herkömmliche, gerade Hubmagnete ausgeführt sind. Dies bedeutet, dass die fest an der Manschette befestigen Tauchkerne (Eisenkerne) der Hubmagnete in herkömmlicher Weise gerade und nicht kreisbogenförmig ausgeführt sind. Ebenso sind die zu den Eisenkernen beweglich geführten bestrombaren Spulen der Elektromagneten gerade ausgeführt, so dass die Hin- und Herbewegung der bestrombaren Spulen nicht entlang eines Kreisbogens, sondern entlang einer Raumachse erfolgt. Die beiden Spulen eines Massenelements sind über Streben statt über eine Kapsel miteinander bewegungsgekoppelt.

Dadurch ist die Vorrichtung weniger kompakt im Vergleich zu der Vorrichtung wie in Figur 3A gezeigt, die Funktionsweise und Wirkung ist ansonsten vergleichbar zu der in Figur 3A gezeigten Ausführungsvariante. Da die Hubmagnete Aktoren darstellen, ist es möglich, die bewegliche Masse aktiv relativ zu den Eisenkernen zu bewegen. Die nachfolgend mit rechts bzw. links bezeichneten Hubmagnete bzw. Spulen entsprechen der Anordnung der Spulen, wie auch in Figur 3A für die Spulen 31a gezeigt ist. Um die Masse relativ zu den Eisenkernen und somit zum Arm, nach links zu bewegen, muss durch die beiden rechten Hubmagnete, d. h. die rechten Spulen, ein elektrischer Strom fließen. Dieser Stromfluss verursacht ein Magnetfeld, welches die beiden rechten Hubmagnete und somit die gesamte bewegliche Masse in Richtung Eisenkerne zieht. Durch die beiden linken Hubmagnete fließt dabei kein Strom. Um die Masse in entgegengesetzter Richtung zu bewegen, wird der elektrische Strom der beiden rechten Hubmagnete abgeschaltet und stattdessen ein elektrischer Strom durch die beiden linken Hubmagnete, d. h. die linken Spulen, geschickt. Das dadurch in den beiden linken Hubmagneten entstehende Magnetfeld zieht die linken Hubmagnete in Richtung der Eisenkerne, und die bewegliche Masse bewegt sich dadurch nach rechts.

Durch abwechselnden Stromfluss durch die beiden linken und die beiden rechten Hubmagnete werden die Eisenkerne und damit der über die Befestigungsmanschette fest mit den Eisenkernen verbundene Arm abwechselnd einmal nach links und dann nach rechts gezogen. Stellt man sich die bewegliche Masse aufgrund ihrer Massenträgheit idealisiert als ortsfest vor, so führt die Relativbewegung zwischen der trägen Masse und den Eisenkernen zu einer Kraftwirkung auf den Arm, welcher mittels der Befestigungsmanschette fest mit den Eisenkernen verbunden ist. Diese Gegenkraft lässt sich nutzen, um der tremorbedingten Zitterbewegung des Arms entgegen zu wirken.

Die Tremorbewegung der Hand kann mittels kommerzieller Beschleunigungssensoren erfasst werden. Geeignet ist beispielsweise der 3-Achs-MEMS-Sensor bma020 der Firma Bosch Sensortec GmbH, Gerhard-Kindler-Straße 8, 72770 Reutlingen, Deutschland. Mögliche Aktoren sind z. B. die Hubmagnete der Serie 42 des Herstellers BLP Components Ltd., Exning Rd, Newmarket, Suffolk CB8 0AX, England. Ein solcher Hubmagnet hat ein Gewicht von 0,213 kg. So ergibt sich inkl. der Streben (Metallschienen) ein Gewicht von etwa 1 kg, welches von den Aktoren bewegt werden muss. Dies ist so groß wie das angenommene Armgewicht.

Da die Tremorbewegung der Hand, gerade im Falle einer Parkinsonerkrankung, ziemlich exakt einer sinusförmigen Bewegung mit konstanter Frequenz entspricht, ist es möglich, mittels der vier Hubmagnete Kräfte auszuüben, die ausreichend groß sind, um eine Masse von 1 kg mit einer Frequenz von 5,56 Hz sinusförmig mit einer Schwingungsamplitude von 1,39 mm zu bewegen. Der Wert 1, 39 mm entspricht einer mittleren Tremoramplitude bei einer Parkinsonerkrankung (siehe [RP Meshack and KE Norman, A randomized controlled trial of the effects of weights on amplitude and frequency of postural hand tremor in people with Parkinson's disease, Clinical Rehabilitation 2002; 16: 481-492]).

Die Magnetkraft der verwendeten Hubmagnete hängt davon ab, wie weit der Eisenkern aus dem Magneten herausgezogen ist. Außerdem hängt die über eine längere Nutzungsdauer erzielbare Kraft davon ab, ob der Magnet im Dauerbetrieb oder mit einer bestimmten relativen Einschaltdauer betrieben wird. Die relative Einschaltdauer gibt z. B. bei zyklisch betriebenen Hubmagneten an, welchen prozentualen Anteil der Gesamtbetriebsdauer (d. h. der Summe aus Ein-Phasen und Aus-Phasen) die Ein-Phasen einnehmen. Je geringer die relative Einschaltdauer gewählt wird, desto höher kann der durch die Magnetwicklung fließende elektrische Strom gewählt werden, ohne dass sich der Hubmagnet während der Nutzungsdauer unzulässig stark erhitzt.

Zum Ausführen der sinusförmigen Bewegung arbeiten stets zwei der vier Hubmagnete parallel, d. h. ziehen zeitgleich in die gleiche Richtung. Die verwendeten Hubmagnete können prinzipbedingt den Eisenkern zwar anziehen, aber nicht mehr abstoßen. Daher muss bei der ersten Halbwelle der Sinusbewegung das erste Hubmagnetenpaar (z. B. die beiden linken Hubmagnete) die Zugkraft aufbringen, und bei der zweiten Halbwelle muss dies das zweite Paar leisten. Alle Hubmagnete sind also nur während 50% der Zeit aktiv. Die relative Einschaltdauer beträgt daher 50%.

Da jeweils zwei Magnete parallel arbeiten, muss jeder Magnet nur 50% des Gesamtgewichts von 1 kg bewegen. Wenn ein Hubmagnet relativ zu seinem Eisenkern eine sinusförmige Bewegung mit einer Amplitude von 1,39 mm ausführen soll, so muss in der Nulllage der Sinusbewegung der Kern bereits um mindestens 1,39 mm von der Position "Kern vollkommen im Magnet" entfernt sein. Nach einer halben Periode der Sinusbewegung hat sich der Kern dann um 2 * 1,39 mm, also um 2,78 mm, aus dem Magneten herausbewegt. In dieser Position ist der Kern am weitesten aus dem Magneten herausgezogen und die Magnetkraft ist minimal. Dem Datenblatt des verwendeten Hubmagneten ist entnehmbar, dass der Hubmagnet bei einer relativen Einschaltdauer von 50% und einem Stroke von 2,78 mm eine Kraft von ca. 1,25 Kilopond (= 1,25*9,81 N = 12,2 N) aufbringen kann. Gemäß der Gleichung Kraft = Masse * Beschleunigung (Formel 3) entspricht die bei einer Amplitude von 1,39 mm und einer Frequenz von 5,56 Hz sinusförmig bewegte Masse (von 0,5 kg, da ein Hubmagnet nur die Hälfte des Gesamtgewichts bewegen muss) einer sinusförmigen Kraft mit einer Amplitude von 0,85 N.

Ein Hubmagnet ist also in der Lage, eine sinusförmige Kraft mit einer Amplitude von 12,2 N aufbringen, obwohl das Bewegen der Massen der Vorrichtung lediglich eine Kraftamplitude von 0,85 N benötigt, um den typischen Handtremor eines Parkinsonpatienten auszugleichen. Selbst wenn die Berechnung mit dem vollen Gewicht von Unterarm und Hand (2,1 kg) erfolgt wäre, würde sich eine nötige Kraft von nur 1,8 N ergeben, die einer Zugkraft des Magneten von 12,2 N gegenübersteht.

Die exemplarisch ausgewählten kommerziell erhältlichen Hubmagnete können also weit unterhalb ihrer Maximalleistung betrieben werden und sind in der Lage, die zum Reduzieren eines Handtremors nötigen Kräfte aufzubringen.

Mit der vorstehend beschriebenen Vorrichtung wurden Testmessungen an einem Probanden durchgeführt.

Ein Beschleunigungssensor wurde auf der inneren und mit dem Arm fix verbundenen Befestigungsmanschette platziert. Seine Signale werden einem Regelalgorithmus zugeführt, welcher berechnet, mit welcher Leistung die Aktoren angesteuert werden müssen. Der Regelalgorithmus wurde mittels der kommerziell erhältlichen Software LabView auf einem Computer implementiert.

Als Aktoren verwendet wurden Zugmagnete der Serie 42 des Herstellers BLP Components Ltd., Exning Rd, Newmarket, Suffolk CB8 0AX, England. Diese wurden mit konstanter Gleichspannung betrieben. Zum Variieren der Zugkraft wurde die Gleichspannung als periodische Folge von Impulsen angelegt, wobei die Periodendauer der Pulse weit unterhalb der Periodendauer der Tremorbewegung lag. Das Verhältnis der Impulsdauer zur Periodendauer, der sogenannte Tastgrad oder duty factor, und damit die effektive Zugkraft des Hubmagneten kann zwischen 0 und 100% variiert werden. Auf Basis der vom Regelalgorithmus gelieferten Werte konnte daher der zeitliche Verlauf der Zugkraft durch Anpassen des zeitlichen Verlaufs des Tastgrads eingestellt werden.

Verwendet wurden vier der genannten Hubmagnete, wobei jeweils zwei Hubmagnete in die gleiche Richtung wirkten. Die Magnetkerne sind alle fest mit der zentral angeordneten eigentlichen Armmanschette (Befestigungsmanschette) verbunden, so dass sich die Hubmagnete bzw. die bestrombaren Spulen relativ zu den Kernen und somit relativ zum Arm bewegen. Ein Hubmagnet hat ein Gewicht von 0,213 kg. So ergibt sich inkl. Querstreben in der Manschette ein Gewicht von etwa 1 kg, welches bewegt werden muss.

Die Zitterbewegung eines Probanden, an dessen Unterarm die erfindungsgemäße Vorrichtung befestigt wurde, wurde während der gesamten Messdauer mittels einer Videokamera aufgenommen. Während der Messdauer wurde von einer zweiten Person ohne Vorankündigung die Spannungsversorgung der Aktoren abwechselnd für ein vorbestimmtes Zeitintervall ein und dann wieder ausgeschaltet.

Für die Zitteramplituden bei ausgeschalteter bzw. eingeschalteter Manschette ergaben sich anhand einer nachfolgenden Videoanalyse folgende Werte: Zitteramplitude der Hand bei angelegter, aber ausgeschalteter Manschette: ca. 5,5 mm. Zitteramplitude der Hand bei angelegter und eingeschalteter Manschette: ca. 2,2 mm. Die Frequenz der Zitterbewegung der Hand lag etwa bei 4,5 Hz. Das Einschalten der Vorrichtung führte somit zu einer Reduzierung der Tremoramplitude um ca. 60%.

Obwohl die Vorrichtung unter Bezugnahme auf bestimmte Ausführungsbeispiele beschrieben worden ist, ist es für einen Fachmann ersichtlich, dass verschiedene Änderungen ausgeführt werden können und Äquivalente als Ersatz verwendet werden können. Zusätzlich können viele Modifikationen ausgeführt werden, ohne den zugehörigen Bereich zu verlassen. Folglich soll die Erfindung nicht auf die offenbarten Ausführungsbeispiele begrenzt sein, sondern durch die beigefügten Patentansprüche.

**Patentansprüche**

1. Am Körper tragbare Vorrichtung (10; 30) zum Reduzieren von Tremor eines Körperteils (1), umfassend
   ein Sensormittel zum Erzeugen eines Signals, das mit einem Tremor des Körperteils (1) korreliert;
   einen Regler, der eingerichtet ist, in Abhängigkeit von dem Signal des Sensormittels eine Stellgröße zu bestimmen; und
   eine Massenverlagerungseinrichtung, umfassend zwei um eine gemeinsame Rotationsachse (R) rotierbar gelagerte Massenelemente (2, 3; 31), die ausgeführt ist, die zwei Massenelemente (2, 3; 31) in Abhängigkeit von der Stellgröße relativ zu dem Körperteil (1) zu bewegen, derart, dass die Bewegung der zwei Massenelemente (2, 3; 31) eine Gegenkraft auf den Körperteil (1) ausübt, die einer durch Tremor verursachten Bewegung des Körperteils (1) entgegenwirkt, **dadurch gekennzeichnet,**

   a) **dass** die zwei Massenelemente (2, 3; 31) im angelegten Zustand der Vorrichtung (10; 30) kreisbogenförmig oder entlang einer Kreisbahn um den Körperteil (1) bewegbar sind; und
   b) **dass** die Stellgröße Rotationsbewegungen der beiden Massenelemente (2, 3; 31) um den Körperteil (1) bewirkt, derart, dass eine aus den Rotationsbewegungen resultierende Zentrifugalkraft (F2, F3) der durch Tremor verursachten Bewegung des Körperteils (1) entgegenwirkt.

2. Vorrichtung (30) nach Anspruch 1, **dadurch gekennzeichnet, dass** die Stellgröße

   a) eine zyklische Hin- und Herbewegung (B1) des ersten der beiden Massenelemente (31) entlang eines ersten Kreisbogens bewirkt und
   b) eine korrespondierende zyklische Hin- und Herbewegung (B2) des zweiten Massenelements (31) im gleichen Drehsinn oder im entgegengesetzten Drehsinn entlang eines zum ersten Kreisbogen gegenüberliegenden zweiten Kreisbogens bewirkt.

3. Vorrichtung (30) nach Anspruch 2, **dadurch gekennzeichnet, dass** jedes der Massenelemente (31) zwei bestrombare, miteinander bewegungsgekoppelte kreisbogenförmige Spulen (31a) aufweist, die relativ zu einem kreisbogenförmigen Tauchkern (32), der starr zum Körperteil angeordnet ist und dessen gegenüberliegende Stirnbereiche (32a) jeweils in den Spuleninnenraum (31c) einer der Spulen eingreifen, bewegbar geführt sind und sich im bestromten Zustand relativ zum Tauchkern (32) bewegen, wobei eine zyklische Hin- und Herbewegung der Spulen (31a) durch ein abwechselndes Bestromen der Spulen (31a) erzeugbar ist.

4. Vorrichtung (30) nach Anspruch 3, **dadurch gekennzeichnet, dass** jedes der Massenelemente (31) eine die beiden Spulen (31a) umgebende kreisbogenförmige Kapsel (31b) umfasst, mit der die Spulen (31a) fest verbunden sind, wobei die Kapsel (31b)

   a) auf dem Tauchkern (32),
   b) auf einer das Körperteil umgebenden Manschette (<34), mit der der Tauchkörper fest verbunden ist, oder
   c) in einem Innenraum (37), der von der Manschette (34) und einer darauf befestigten Außenverkleidung (36) gebildet wird, relativ zum Tauchkern (32) bewegbar geführt ist.

5. Vorrichtung (30) nach einem der Ansprüche 2 bis 4, **dadurch gekennzeichnet, dass**

a) die zwei um die gemeinsame Rotationsachse rotierbar gelagerten Massenelemente im Innenraum eines ersten Ringkörpers oder einer ersten Teilmanschette vorgesehen sind und

b) ein zweiter im wesentlichen baugleicher Ringkörper bzw. zweite Teilmanschette vorgesehen ist, in dem zwei weitere um die gemeinsame Rotationsachse rotierbar gelagerte Massenelemente vorgesehen sind, wobei der zweite Ringkörper gedreht, vorzugsweise um 90°, zum ersten Ringkörper angeordnet ist, so dass sich die Raumrichtung der von der Bewegung der Massenelemente des ersten Ringkörpers erzeugten Gegenkraft von derjenigen des zweiten Ringkörpers unterscheidet.

6. Vorrichtung (10) nach Anspruch 1, **dadurch gekennzeichnet, dass** die Massenverlagerungseinrichtung zwei drehbar gelagerte Ringkörper (4, 5) umfasst, an denen jeweils eines der Massenelemente (2, 3) ortsfest befestigt ist.

7. Vorrichtung (10) nach Anspruch 6, **dadurch gekennzeichnet, dass**

a) die Vorrichtung ein Gehäuse (8, 18) umfasst, das zur Umgreifung des Körperteils (1) ringförmig ausgebildet ist, wobei an einer dem Körperteil (1) zugewandten Außenfläche (14) des Gehäuses (8) ein aufpumpbares Polster (15) angeordnet ist, eine oder mehrere Aktoren (13) zum Aufpumpen des Polsters (15) in dem Gehäuse (8) angeordnet sind und das Gehäuse (8) durch Aufpumpen des Polsters (15) ortsfest am Körperteil (1) befestigbar ist; und/oder

b) dass die Ringkörper (4, 5) mittels Kugellager (12) im Innern (23) des Gehäuses (8) unabhängig voneinander um den Körperteil (1) drehbar gelagert sind; und/oder cc) dass die Massenverlagerungseinrichtung zwei Motoren umfasst, die zum Drehantrieb der Ringkörper (4, 5) jeweils mit einem der Ringkörper (4, 5) in Wirkverbindung stehen.

8. Vorrichtung nach Anspruch 1, **dadurch gekennzeichnet,**

a) **dass** die Massenverlagerungseinrichtung zwei ortsfest zum Körperteil (1) anordenbare Ringkörper umfasst, wobei jeweils eines der Massenelemente auf einem der beiden Ringkörper entlang eines Kreisbogens oder einer Umfangskreisbogenlinie verfahrbar gelagert ist; oder

b) **dass** die Massenverlagerungseinrichtung einen ortsfest zum Körperteil anordenbaren Ringkörper umfasst, auf dessen äußerer Mantelfläche die beiden Massenelemente axial versetzt zueinander verfahrbar gelagert sind, wobei die Verfahrbewegung der Massenelemente entlang eines Kreisbogens oder entlang einer Kreisbahn erfolgt.

9. Vorrichtung nach einem der Ansprüche 6 bis 8, **dadurch gekennzeichnet, dass** jedes der beiden Massenelemente zwei Teilmassen umfasst, die auf einer Umfangslinie des Ringkörpers des jeweiligen Massenelements angeordnet sind und deren Abstand zueinander variierbar ist.

10. Vorrichtung nach einem der vorhergehenden Ansprüche 1 bis 9, **dadurch gekennzeichnet, dass** der Regler eingerichtet ist, eine erste Stellgröße zu bestimmen, die jeweils eine Kreisbewegung der beiden Massenelemente (2, 3) mit vorzugsweise gleicher Winkelgeschwindigkeit, aber entgegengesetzter Drehrichtung (6, 7) bewirkt.

11. Vorrichtung nach einem der vorhergehenden Ansprüche 1 bis 10, **dadurch gekennzeichnet, dass** der Regler eingerichtet ist, eine zweite Stellgröße zu bestimmen, die eine zyklische Hin- und Herbewegung der beiden Massenelemente (2, 3; 31) jeweils entlang eines Kreisbogens, vorzugsweise Halbkreises, mit vorzugsweise gleicher Winkelgeschwindigkeit, aber entgegengesetzter Drehrichtung bewirkt.

12. Vorrichtung nach einem der vorhergehenden Ansprüche 1 bis 11, **dadurch gekennzeichnet, dass** der Regler eingerichtet ist, zur Reduzierung einer rotativen Zitterbewegung eine dritte Stellgröße zu bestimmen, die eine zyklische Hin- und Herbewegung der beiden Massenelemente (2, 3; 31) mit vorzugsweise gleicher Winkelgeschwindigkeit in die gleiche Drehrichtung bewirkt.

13. Vorrichtung nach einem der vorhergehenden Ansprüche, **dadurch gekennzeichnet, dass** der Regler oder das Sensormittel

a) einen ersten Filter umfasst, der Frequenzen oberhalb eines oberen Grenzwerts herausfiltert; und/oder

b) einen zweiten Filter umfasst, der Frequenzen unterhalb eines unteren Grenzwerts herausfiltert; und/oder

c) einen dritten Filter umfasst, welcher als adaptiver Filter ausgeführt ist und eine mögliche zeitliche Änderung der Tremorfrequenz berücksichtigt; und/oder

d) einen vierten Filter umfasst, welcher zum Berechnen der tremorbedingten Beschleunigungen des Körperteils einen rekursiven Algorithmus verwendet.

14. Vorrichtung nach einem der vorhergehenden Ansprüche, **dadurch gekennzeichnet,**

a) **dass** der Regler als Regler mit einem proportionalen Verhalten oder einem Regler mit integralem Verhalten oder einem Regler mit differentialem Verhalten oder einer Kombination aus mehreren der genannten Reglertypen ausgeführt ist; und/oder
b) **dass** der Körperteil eine Gliedmaße ist, vorzugsweise ein Oberarm, ein Unterarm oder ein Handgelenk; und/oder;
c) **dass** die Vorrichtung manschettenförmig oder stulpenförmig ausgeführt ist; und/oder
d) **dass** die Vorrichtung Befestigungsmittel zur Befestigung am Körperteil, insbesondere einen Klettverschluss, umfasst;
und/oder
e) **dass** die Vorrichtung eine Energieversorgungseinheit umfasst; und/oder
f) **dass** die Masse der zwei Massenelemente (2, 3; 31) veränderbar ist.


**Claims**

1. Device (10; 30) wearable on the body for reducing tremor of a body part (1), comprising
a sensor means for generating a signal that correlates with a tremor of the body part (1);
a regulator which is configured, dependent upon the signal of the sensor means, to determine a manipulated variable; and
a mass displacement apparatus comprising two mass elements (2, 3; 31) which are mounted to be rotatable about a common rotation axis (R), which is designed to move the two mass elements (2, 3; 31) relative to the body part (1) dependent upon the manipulated variable, such that the movement of the two mass elements (2, 3; 31) exerts a counterforce on the body part (1), which force counteracts a movement caused by tremor of the body part (1), **characterised in that**

a) in the applied state of the device (10; 30), the two mass elements (2, 3; 31) are movable in the form of a circular arc or along a circular path about the body part (1); and
b) **in that** the manipulated variable causes rotational movements of the two mass elements (2, 3; 31) about the body part (1) such that a centrifugal force (F2, F3) resulting from the rotational movements counteracts the movement of the body part (1) caused by tremor.

2. Device (30) according to claim 1, **characterised in that** the manipulated variable

a) causes a cyclical back and forth movement (B1) of the first of the two mass elements (31) along a first circular arc, and
b) causes a corresponding cyclical back and forth movement (B2) of the second mass element (31) in the same rotational sense or in the opposite rotational sense along a second circular arc situated opposite the first circular arc.

3. Device (30) according to claim 2, **characterised in that** each of the mass elements (31) has two circular arc-shaped coils (31a) motion-coupled to one another, to which current can be applied, which are guided to be movable relative to a circular arc-shaped plunger core (32) which is rigidly arranged relative to the body part, and the opposite end regions (32a) of which each engage in the coil interior (31c) of one of the coils and, in the current-carrying state, move relative to the plunger core (32), wherein a cyclical back and forth movement of the coils (31a) is generatable by means of an alternating application of current to the coils (31a).

4. Device (30) according to claim 3, **characterised in that** each of the mass elements (31) comprises a circular arc-shaped capsule (31b) surrounding the two coils (31a), to which the coils (31a) are firmly attached, wherein the capsule (31b)

a) is firmly attached to the plunger core (32),
b) is firmly attached to a sleeve (34) which surrounds the body part and to which the plunger body is firmly attached, or

c) is guided to be movable relative to the plunger core (32) in an interior space (37) which is formed by the sleeve (34) and an outer covering (36) fastened thereto.

5. Device (30) as claimed in one of the claims 2 to 4, **characterised in that**

   a) the two mass elements mounted rotatably about the common rotation axis are provided in the interior of a first ring body or a first partial sleeve, and
   b) a second substantially identically constructed ring body or a second partial sleeve is provided in which two further mass elements which are mounted rotatably about the common rotation axis are provided, wherein the second ring body is arranged rotated, preferably by 90°, relative to the first ring body so that the spatial direction of the counterforce generated by the movement of the mass elements of the first ring body differs from that of the second ring body.

6. Device (10) according to claim 1, **characterised in that** the mass displacement apparatus comprises two rotatably mounted ring bodies (4, 5) to each of which one of the mass elements (2, 3) is immovably fastened.

7. Device (10) according to claim 6, **characterised in that**

   a) the device comprises a housing (8, 18) which is configured annular to encompass the body part (1), wherein on an outer surface (14) of the housing (8) facing the body part (1), an inflatable cushion (15) is arranged, one or more actuators (13) for inflating the cushion (15) are arranged in the housing (8) and the housing (8) is immovably fastenable on the body part (1) by inflating the cushion (15); and/or
   b) **in that** the ring bodies (4, 5) are mounted by means of ball bearings (12) in the interior (23) of the housing (8), to be rotatable independently of one another about the body part (1); and/or
   c) **in that** the mass displacement apparatus comprises two motors which, for rotary driving of the ring body (4, 5) are each in operative engagement with one of the ring bodies (4, 5).

8. Device according to claim 1, **characterised in that**

   a) the mass displacement apparatus comprises two ring bodies which are arrangeable immovably relative to the body part (1), wherein one of the mass elements is mounted, in each case, on one of the two ring bodies movable along a circular arc or a circumferential circular arc line; or
   b) **in that** the mass displacement apparatus comprises a ring body which is arrangeable immovably relative to the body part, on the outer envelope surface of said ring body the two mass elements are mounted axially offset from one another, wherein the displacement movement of the mass elements takes place along a circular arc or along a circular path.

9. Device according to one of the claims 6 to 8, **characterised in that** each of the two mass elements comprises two partial masses which are arranged on a circumferential line of the ring body of the respective mass element and the spacing thereof from one another is variable.

10. Device according to one of the preceding claims 1 to 9, **characterised in that** the regulator is configured to determine a first manipulated variable which causes a circular movement of each of the two mass elements (2, 3), preferably with the same angular velocity but opposite rotation directions (6, 7).

11. Device according to one of the preceding claims 1 to 10, **characterised in that** the regulator is configured to determine a second manipulated variable which causes a cyclical back and forth movement of the two mass elements (2, 3; 31), respectively along a circular arc, preferably a semicircle, preferably with the same angular velocity but opposite rotation directions.

12. Device according to one of the preceding claims 1 to 11, **characterised in that** the regulator is configured, in order to reduce a rotational tremor movement, to determine a third manipulated variable which causes a cyclical back and forth movement of the two mass elements (2, 3; 31), preferably with the same angular velocity in the same rotational direction.

13. Device according to one of the preceding claims, **characterised in that** the regulator or the sensor means

   a) comprises a first filter which filters out frequencies above an upper limit value; and/or

b) comprises a second filter which filters out frequencies below a lower limit value; and/or

c) comprises a third filter which is designed as an adaptive filter and takes account of a possible temporal change in the tremor frequency; and/or

d) comprises a fourth filter which uses a recursive algorithm for calculating the tremor-related accelerations of the body part.

**14.** Device according to one of the preceding claims, **characterised in that**

a) the regulator is designed as a regulator with proportional behaviour or a regulator with integral behaviour or a regulator with derivative behaviour or a combination of a plurality of the regulator types mentioned; and/or

b) **in that** the body part is a limb, preferably an upper arm, a forearm or a wrist; and/or

c) **in that** the device is designed sleeve-shaped or cuff-shaped; and/or

d) **in that** the device comprises fastening means for fastening to the body part, in particular a hook-and-loop fastening; and/or

e) **in that** the device comprises an energy supply unit; and/or

f) **in that** the mass of the two mass elements (2, 3; 31) is changeable.

**Revendications**

**1.** Dispositif (10 ; 30) pouvant être porté sur le corps pour réduire le tremblement d'une partie corporelle (1), comprenant un moyen de capteur pour générer un signal, qui est en corrélation avec un tremblement de la partie corporelle (1) ; un régulateur, qui est mis au point pour définir en fonction du signal du moyen de capteur une grandeur de réglage ; et un système de déplacement de masse comprenant deux éléments de masse (2, 3 ; 31) montés de manière à pouvoir tourner autour d'un axe de rotation (R) commun, qui est réalisé pour déplacer les deux éléments de masse (2, 3 ; 31) en fonction de la grandeur de réglage par rapport à la partie corporelle (1) de telle manière que le déplacement des deux éléments de masse (2, 3 ; 31) exerce une contre-force sur la partie corporelle (1), qui contrecarre un mouvement, provoqué par le tremblement, de la partie corporelle (1), **caractérisé en ce**

a) **que** les deux éléments de masse (2, 3 ; 31) peuvent être déplacés en forme d'arc de cercle ou le long d'une trajectoire circulaire autour de la partie corporelle (1) dans l'état placé du dispositif (10 ; 30) ; et

b) **que** la grandeur de réglage entraîne des mouvements de rotation des deux éléments de masse (2, 3 ; 31) autour de la partie corporelle (1) de telle manière qu'une force centrifuge (F2, F3) résultant des mouvements de rotation contrecarre le mouvement, provoqué par le tremblement, de la partie corporelle (1).

**2.** Dispositif (30) selon la revendication 1, **caractérisé en ce que** la grandeur de réglage

a) entraîne un mouvement cyclique de va-et-vient (B1) du premier des deux éléments de masse (31) le long d'un premier arc de cercle, et

b) entraîne un mouvement cyclique de va-et-vient (B2) correspondant du second élément de masse (31) dans le même sens de rotation ou dans le sens de rotation opposé le long d'un deuxième arc de cercle faisant face au premier arc de cercle.

**3.** Dispositif (30) selon la revendication 2, **caractérisé en ce que** chacun des éléments de masse (31) présente deux bobines (31a) en forme d'arc de cercle couplées en mouvement l'une à l'autre, pouvant être alimentées en courant, qui sont guidées de manière à pouvoir être déplacées par rapport à un noyau d'immersion (32) en forme d'arc de cercle, qui est disposé de manière rigide par rapport à la partie corporelle et dont les zones frontales (32a) se faisant face viennent en prise respectivement avec l'espace intérieur de bobine (31c) d'une des bobines, et se déplacent par rapport au noyau plongeur (32) dans l'état alimenté en courant, dans lequel un mouvement cyclique de va-et-vient des bobines (31a) peut être généré par une alimentation en courant alternative des bobines (31a).

**4.** Dispositif (30) selon la revendication 3, **caractérisé en ce que** chacun des éléments de masse (31) comprend une capsule (31b) en forme d'arc de cercle entourant les deux bobines (31a), à laquelle les bobines (31a) sont reliées de manière solidaire, dans lequel la capsule (31b) est guidée de manière à pouvoir être déplacée par rapport au noyau plongeur (32)

a) sur le noyau plongeur (32),

b) sur une manchette (34) entourant la partie corporelle, à laquelle le corps plongeur est relié de manière solidaire, ou

c) dans un espace intérieur (37), qui est formé par la manchette (34) et un habillage extérieur (36) fixé sur celle-ci.

**5.** Dispositif (30) selon l'une quelconque des revendications 2 à 4, **caractérisé en ce que**

a) les deux éléments de masse montés de manière à pouvoir tourner autour de l'axe de rotation commun sont prévus dans l'espace intérieur d'un premier corps annulaire ou d'une première manchette partielle, et

b) un deuxième corps annulaire à structure sensiblement identique ou une deuxième manchette partielle est prévu ou prévue, dans lequel ou laquelle deux autres éléments de masse montés de manière à pouvoir tourner autour de l'axe de rotation commun sont prévus, dans lequel le deuxième corps annulaire est disposé de manière tournée, de préférence de 90°, par rapport au premier corps annulaire, de sorte que la direction spatiale de la contre-force générée par le mouvement des éléments de masse du premier corps annulaire se distingue de celle du deuxième corps annulaire.

**6.** Dispositif (10) selon la revendication 1, **caractérisé en ce que** le système de déplacement de masse comprend deux corps annulaires (4, 5) montés de manière à pouvoir tourner, sur lesquels respectivement un des éléments de masse (2, 3) est fixé de manière stationnaire.

**7.** Dispositif (10) selon la revendication 6, **caractérisé en ce que**

a) le dispositif comprend un boîtier (8, 18), qui est réalisé pour entourer de manière à présenter une forme annulaire la partie corporelle (1), dans lequel un rembourrage (15) gonflable est disposé sur une surface extérieure (14), tournée vers la partie corporelle (1), du boîtier (8), un ou plusieurs actionneurs (13) pour gonfler le rembourrage (15) sont disposés dans le boîtier (8) et le boîtier (8) peut être fixé de manière stationnaire sur la partie corporelle (1) en gonflant le rembourrage (15) ; et/ou

b) que les corps annulaires (4, 5) sont montés de manière à pouvoir tourner autour de la partie corporelle (1) indépendamment les uns des autres au moyen de roulements (12) dans l'intérieur (23) du boîtier (8) ; et/ou

c) que le système de déplacement de masse comprend deux moteurs, qui coopèrent respectivement avec un des corps annulaires (4, 5) pour l'entraînement en rotation des corps annulaires (4, 5).

**8.** Dispositif selon la revendication 1, **caractérisé en ce**

a) **que** le système de déplacement de masse comprend deux corps annulaires pouvant être disposés de manière stationnaire par rapport à la partie corporelle (1), dans lequel respectivement un des éléments de masse est monté de manière à pouvoir être déplacé sur un des deux corps annulaires le long d'un arc de cercle ou une ligne d'arc de cercle périphérique ; ou

b) **que** le système de déplacement de masse comprend un corps annulaire pouvant être disposé de manière stationnaire par rapport à la partie corporelle, sur la surface enveloppante extérieure duquel les deux éléments de masse sont montés de manière à pouvoir être déplacés de manière décalée l'un par rapport à l'autre, dans lequel le mouvement de déplacement des éléments de masse a lieu le long d'un arc de cercle ou le long d'une trajectoire circulaire.

**9.** Dispositif selon l'une quelconque des revendications 6 à 8, **caractérisé en ce que** chacun des deux éléments de masse comprend deux masses partielles, qui sont disposées sur une ligne périphérique du corps annulaire de l'élément de masse respectif et dont la distance l'une par rapport à l'autre peut varier.

**10.** Dispositif selon l'une quelconque des revendications précédentes 1 à 9, **caractérisé en ce que** le régulateur est mis au point pour définir une première grandeur de réglage, qui entraîne respectivement un mouvement circulaire des deux éléments de masse (2, 3) avec de préférence une vitesse angulaire identique, toutefois avec un sens de rotation (6, 7) opposé.

**11.** Dispositif selon l'une quelconque des revendications précédentes 1 à 10, **caractérisé en ce que** le régulateur est mis au point pour définir une deuxième grandeur de réglage, qui entraîne un mouvement cyclique de va-et-vient des deux éléments de masse (2, 3 ; 31) respectivement le long d'un arc de cercle, de préférence d'un demi-cercle, avec une vitesse angulaire de préférence identique, toutefois avec un sens de rotation opposé.

**12.** Dispositif selon l'une quelconque des revendications précédentes 1 à 11, **caractérisé en ce que** le régulateur est mis au point pour définir, afin de réduire un mouvement de tremblement rotatif, une troisième grandeur de réglage, qui entraîne un mouvement cyclique de va-et-vient des deux éléments de masse (2, 3 ; 31) avec une vitesse angulaire de préférence identique dans le même sens de rotation.

**13.** Dispositif selon l'une quelconque des revendications précédentes, **caractérisé en ce que** le régulateur ou le moyen de capteur

a) comprend un premier filtre, qui filtre des fréquences supérieures à une valeur de limite supérieure ; et/ou
b) comprend un deuxième filtre, qui filtre des fréquences inférieures à une valeur de limite inférieure ; et/ou
c) comprend un troisième filtre, lequel est réalisé en tant que filtre adaptatif et tient compte d'une modification dans le temps possible de la fréquence de tremblement ; et/ou
d) comprend un quatrième filtre, lequel utilise un algorithme récursif pour calculer les accélérations, liées au tremblement, de la partie corporelle.

**14.** Dispositif selon l'une quelconque des revendications précédentes, **caractérisé en ce**

a) **que** le régulateur est réalisé en tant que régulateur avec un comportement proportionnel ou un régulateur avec un comportement intégral ou un régulateur avec un comportement différentiel ou une combinaison composée de plusieurs types de régulateur mentionnés ; et/ou
b) **que** la partie corporelle est un membre, de préférence un haut de bras, un avant-bras ou une articulation de la main ; et/ou
c) **que** le dispositif est réalisé en forme de manchette ou en forme de revers ; et/ou
d) **que** le dispositif comprend des moyens de fixation pour être fixés sur la partie corporelle, en particulier une fermeture autoagrippante ; et/ou
e) **que** le dispositif comprend une unité d'alimentation en énergie ; et/ou
f) **que** la masse des deux éléments de masse (2, 3 ; 31) peut être modifiée.

FIG. 1

FIG. 2

<u>10</u>

# FIG. 3

FIG. 3A

30

B1

32
33
32
32a
31a
31c

32
31
31a
31c
31b

34a

32a

11
37
34
35
36

37

31

B2

FIG. 3B

30

B1

31

31b

31b

31

B2

FIG. 3C

FIG. 3D

FIG. 3E

# FIG. 3F

FIG. 4

<u>40</u>

# FIG. 4a

40a

FIG. 5

# FIG. 6

| | |
|---|---|
| Messe Tremorbewegung des Körperteils | S1 |
| Vergleiche aktueller Messwert mit vorherigem Messwert/Sollwert | S2 |
| Ermittle Stellgröße | S3 |
| Bewege Masse gemäß Stellgröße | S4 |

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2003006357 A1
- WO 0180710 A2
- US 6234045 B1

**In der Beschreibung aufgeführte Nicht-Patentliteratur**

- **RP MESHACK ; KE NORMAN.** A randomized controlled trial of the effects of weights on amplitude and frequency of postural hand tremor in people with Parkinson's disease. *Clinical Rehabilitation,* 2002, vol. 16, 481-492
- **DEUSCHL, G.** Leitlinien für Diagnostik und Therapie in der Neurologie. Thieme Verlag, September 2012
- Leitlinien für Diagnostik und Therapie. **DEUSCHL, G.** Neurologie. Thieme Verlag, September 2012
- **KALYANA C. VELUVOLU ; WEI TECH ANG.** Estimation of Physiological Tremor from Accelerometers for Real-Time Applications. *Sensors,* 2011, vol. 11, 3020-3036
- **W. T. LATT ; U-X. TAN ; K. C. VELUVOLU ; C. Y. SHEE ; W. T. ANG.** Physiological Tremor Sensing using only Accelerometers for Realtime Compensation. *Proceedings of the 2008 IEEE International Conference on Robotics and Biomimetics Bangkok,* 21. Februar 2009
- **ELBLE et al.** Factors Influencing the Amplitude and Frequency of Essential Tremor. *Movement Disorders,* 1994, vol. 9 (6), 589-596